(19) Europäisches Patentamt
European Patent Office
Office européen des brevets

(11) Veröffentlichungsnummer: **0 569 457 B1**

(12) # EUROPÄISCHE PATENTSCHRIFT

(45) Veröffentlichungstag der Patentschrift: **14.06.95**

(51) Int. Cl.[6]: **C07D 513/04**, A61K 31/425, //(C07D513/04,277:00,209:00)

(21) Anmeldenummer: **92904272.9**

(22) Anmeldetag: **28.01.92**

(86) Internationale Anmeldenummer: **PCT/EP92/00172**

(87) Internationale Veröffentlichungsnummer: **WO 92/13863 (20.08.92 92/22)**

(54) **VERWENDUNG VON THIAZOLO-[2,3-A]ISOINDOL-DERIVATEN ALS ANTIVIRALE ARZNEIMITTEL UND NEUE THIAZOLO- [2,3-A]ISOINDOL-DERIVATE.**

(30) Priorität: **02.02.91 DE 4103177**

(43) Veröffentlichungstag der Anmeldung: **18.11.93 Patentblatt 93/46**

(45) Bekanntmachung des Hinweises auf die Patenterteilung: **14.06.95 Patentblatt 95/24**

(84) Benannte Vertragsstaaten: **AT BE CH DE DK ES FR GB GR IT LI LU NL SE**

(56) Entgegenhaltungen:
**CH-A- 469 733**
**GB-A- 1 039 117**

**JOURNAL OF ORGANIC CHEMISTRY Vol. 30, No. 5 1965, EASTON US pages 1506- 1508 R.G. HISKEY ET AL:" Azomethine chemistry. IV. Chemistry of fused thiazolidines."**

(73) Patentinhaber: **BOEHRINGER MANNHEIM GMBH**

**D-68298 Mannheim (DE)**

(72) Erfinder: **ZILCH, Harald**
**Alsenweg 24**
**D-6800 Mannheim 31 (DE)**
Erfinder: **LEINERT, Herbert**
**Essigkamm 11**
**D-6148 Heppenheim (DE)**
Erfinder: **MERTENS, Alfred**
**Beethovenstr. 20**
**D-6905 Schriesheim (DE)**
Erfinder: **SCHAEFER, Wolfgang**
**Feldbergstr. 60**
**D-6800 Mannheim 1 (DE)**
Erfinder: **POLL, Thomas**
**Gambrinusstr. 4A**
**D-6800 Mannheim 31 (DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services
(3.10/3.09/3.3.3)

**JOURNAL OF THE AMERICAN CHEMICAL SOCIETY Vol.80, No. 3 1958k GASTON PA US pages 702- 707 G.L. OLIVER ET AL:" Preparation of thiazolidines and related compounds: Lactams and lactamidines ."**

**LIEBIGS ANNALEN DER CHEMIE. No. 4, 1985, WEINHEIM DE pages 657 - 672; L. SOMOGYI ET AL : " Beiträge zur Chemie des Chiralitätszentrums X-CHR-Y cyclischer N, N-und N, S-acetate."**

**JOURNAL OF MEDICINAL CHEMISTRY Vol. 21, No. 1, 1978, WASHINGTON US pages 82-87; M.R. HARNDEN ET AL: "Thiazolinone analogues of indolmycin with antiviral and antibacterial activity"**

Erfinder: **KÖNIG, Bernhard**
**Dürrbergstr. 28**
**D-8137 Berg 3 (DE)**
Erfinder: **LESER, Ulrike**
**Stiftsbogen 64**
**D-8000 München 70 (DE)**

(74) Vertreter: **Mink, Reinhold, Dr. et al**
**c/o Boehringer Mannheim GmbH,**
**Patentabteilung,**
**Sandhoferstrasse 116**
**D-68298 Mannheim (DE)**

**Beschreibung**

Die vorliegende Erfindung bezieht sich auf die Verwendung von Thiazolo-[2,3-a]isoindol-Derivaten zur Herstellung von antiviralen Arzneimitteln und auf neue Thiazolo-[2,3-a]-isoindol-Derivate.

Die Erfindung betrifft insbesondere die Verwendung von Thiazolo-[2,3-a]isoindol-Derivaten der allgemeinen Formel I

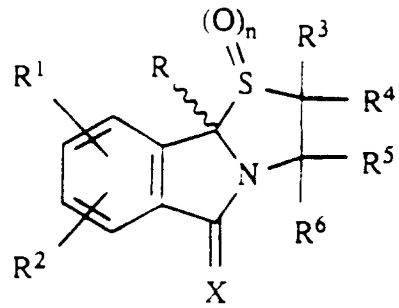

(I),

zur Herstellung von Arzneimitteln zur Behandlung von viralen oder retroviralen Infektionen, wobei

- X ein Sauerstoff- oder Schwefelatom, die Iminogruppe =NH oder eine N-$C_1$-$C_5$-Alkyliminogruppe sein kann,
- n gleich 0, 1 oder 2 ist,
- R ein Wasserstoffatom, einen geradkettigen oder verzweigten, gesättigten oder ungesättigten aliphatischen Rest mit 1-9 C-Atomen, der durch Phenyl substituiert sein kann, oder einen $C_1$-$C_6$-Alkoxy-$C_1$-$C_6$-alkyl- oder $C_1$-$C_6$-Alkylmercapto-$C_1$-$C_6$-alkylrest bedeutet, oder einen Phenylring bedeutet, der gegebenenfalls ein- oder mehrfach substituiert ist durch $C_1$-$C_6$-Alkyl, $C_1$-$C_6$-Alkoxy, $C_1$-$C_6$-Alkylmercapto, $C_1$-$C_6$-Alkylsulfinyl, $C_1$-$C_6$-Alkylsulfonyl, $C_2$-$C_6$-Alkenyl, $C_2$-$C_6$-Alkinyl, $C_2$-$C_6$-Alkenyloxy, $C_2$-$C_6$-Alkenylmercapto, $C_2$-$C_6$-Alkinyloxy, $C_2$-$C_6$-Alkinylmercapto, Amino, $C_1$-$C_6$-Alkylamino, Di-$C_1$-$C_6$-alkylamino, $C_1$-$C_6$-Alkylcarbonylamino, $C_1$-$C_6$-Alkylaminocarbonyl, $C_1$-$C_6$-Alkoxycarbonyl, Aminocarbonyl, Hydroxy, Benzyloxy, Phenylmercapto, Phenyloxy, Nitro, Cyano, Halogen, Trifluormethyl, Azido, Formylamino, Carboxy oder Phenyl, oder
  einen mono-, bi- oder tricyclischen carbocyclischen Ring mit 7-15 C-Atomen oder ein heterocyclisches mono-, bi- oder tricyclisches Ringsystem mit jeweils 5 oder 6 Ringatomen bedeutet und pro Ringsystem 1-4 bzw. 1-5 Heteroatome enthalten sein können, wobei die Heteroatome Stickstoff, Schwefel oder Sauerstoff sind, und diese Ringe durch $C_1$-$C_6$-Alkyl, $C_1$-$C_6$-Alkoxy, Nitro, Amino oder Halogen substituiert sein können,
- $R^1$ ein Wasserstoffatom, einen geradkettigen oder verzweigten, gesättigten oder ungesättigten aliphatischen Rest mit 1-6 C-Atomen oder $C_1$-$C_6$-Alkoxy, $C_1$-$C_6$-Alkylmercapto, $C_1$-$C_6$-Alkylsulfinyl, $C_1$-$C_6$-Alkylsulfonyl, Amino, $C_1$-$C_6$-Alkylamino, Di-$C_1$-$C_6$-Alkylamino, Sulfonamido, $C_1$-$C_6$-Alkoxycarbonyl, Carboxy, Halogen, Hydroxy, Nitro, Cyano, Azido, Phenyl oder Benzyloxy bedeutet,
- $R^2$ die gleiche Bedeutung hat wie $R^1$, wobei die Reste $R^1$ und $R^2$ unabhangig voneinander gleich oder verschieden sein konnen,
- $R^3$ Wasserstoff, $C_1$-$C_6$-Alkyl, $C_1$-$C_6$-Alkoxy, $C_1$-$C_6$-Alkylmercapto, Amino, $C_1$-$C_6$-Alkylamino, Di-$C_1$-$C_6$-Alkylamino, Aminocarbonyl, $C_1$-$C_6$-Alkylaminocarbonyl, Di-$C_1$-$C_6$-alkylaminocarbonyl, Morpholinocarbonyl, Halogen, Cyano, Hydroxy, Carboxy, $C_1$-$C_6$-Alkoxycarbonyl, Aryloxycarbonyl, Hetaryloxycarbonyl, Aryl-$C_1$-$C_6$-alkoxycarbonyl, Hetaryl-$C_1$-$C_6$-alkoxycarbonyl, $C_1$-$C_6$-Alkoxy-$C_1$-$C_6$-alkoxycarbonyl oder Hydroxy-$C_1$-$C_6$-alkoxycarbonyl bedeutet, wobei die Aryl- oder Hetarylreste jeweils substituiert sein können durch $C_1$-$C_6$-Alkyl, $C_1$-$C_6$-Alkoxy oder Halogen,
- $R^4$, $R^5$, $R^6$ die gleiche Bedeutung wie $R^3$ haben, wobei die Reste $R^3$, $R^4$, $R^5$ und $R^6$ unabhängig voneinander gleich oder verschieden sein können,

sowie deren Tautomere, Enantiomere, Diastereomere und physiologisch verträgliche Salze,
mit der Maßgabe, daß für den Fall, daß $R^1$, $R^2$, $R^3$, $R^4$, $R^5$ und $R^6$ gleichzeitig Wasserstoff, n die Zahlen 0 bzw. 1 und X ein Sauerstoffatom bedeuten, der Rest R nicht Wasserstoff, eine aliphatische Gruppe mit 1-7 C-Atomen, die durch Phenyl substituiert sein kann, oder Phenyl, das ein- oder mehrfach substituiert ist

durch $C_1$-$C_4$-Alkyl, $C_1$-$C_4$-Alkoxy, Hydroxy, Trifluormethyl, Methylsulfonyl oder Halogen, bedeuten kann.

Gegenstand der vorliegenden Erfindung sind auch neue Thiazolo[2,3-a]isoindole der Formel I, in der R einen heterocyclischen mono-, bi- oder tricyclischen Ring mit jeweils 5 oder 6 Ringatomen bedeutet und pro Ringsystem 1-4 bzw. 1-5 Heteroatome enthalten sein können, wobei die Heteroatome Sauerstoff, Schwefel oder Stickstoff sein können, und diese Ringe durch $C_1$-$C_6$-Alkyl, $C_1$-$C_6$-Alkoxy, Nitro, Amino oder Halogen substituiert sein können.

Thiazolo-[2,3-a]isoindole der Formel I, in der X ein Sauerstoffatom, n die Zahlen 0 und 1, $R_1$-$R_6$ jeweils Wasserstoff und R ein Wasserstoffatom oder einen aliphatischen Rest mit 1-7 C-Atomen, der durch Phenyl substituiert sein kann, oder R einen Phenylring, der ein- oder mehrfach substituiert sein kann durch Alkyl, Alkoxy, Hydroxy, Trifluormethyl, Methylsulfonyl oder Halogen, bedeuten kann, sind aus der früheren deutschen Patentanmeldung P 40 35 809.7 (WO 92/08457) als antivirale Arzneimittel bekannt.

Ferner sind einzelne Verbindungen der Formel I, in der R ein Wasserstoffatom darstellt, aus J. Org. Chem. 30, 1965, 1506-1508; J. Am. Chem. Soc. 80, 1958, 702-707 und Liebigs Ann. Chem. 4, 1985, 657-672 bekannt. Verbindungen mit X = 0 und R = Phenyl oder Naphthyl sind aus GB 1,039,117 als Arzneimittel mit anti-inflammatorischer, anticonvulsiver und analgetischer Wirkung beschrieben.

Der vorliegenden Erfindung lag die Aufgabe zugrunde, eine weitere medizinische Indikation für bekannte Verbindungen der Formel I zu finden und neue Thiazolo-[2,3-a]isoindole mit antiviraler Wirksamkeit zur Verfügung zu stellen. Diese Aufgabe wird durch die in den Ansprüchen gekennzeichneten Merkmale gelöst.

Die Verbindungen der Formel I weisen wertvolle pharmakologische Eigenschaften auf. Insbesondere eignen sie sich zur Therapie und Prophylaxe von Infektionen, die durch DNA-Viren wie z.B. das Herpes-Simplex-Virus, das Zytomegalie-Virus, Papilloma-Viren, das Varicella-Zoster-Virus oder Epstein-Barr-Virus oder RNA-Viren wie Toga-Viren oder insbesondere Retroviren wie die Onko-Viren HTLV-I und II, sowie die Lentiviren Visna und Humanes-Immunschwäche-Virus HIV-1 und - 2, verursacht werden.

Besonders geeignet erscheinen die Verbindungen der Formel I zur Behandlung der klinischen Manifestationen der retroviralen HIV-Infektion beim Menschen, wie der anhaltenden, generalisierten Lymphadenopathie (PGL), dem fortgeschrittenen Stadium des AIDS-verwandten Komplex (ARC) und dem klinischen Vollbild von AIDS.

Die erfindungsgemäßen Verbindungen der allgemeinen Formel I besitzen eine ausgeprägte antivirale Wirkung und eignen sich insbesondere zur Behandlung von viralen bzw. retro-viralen Infektionen. Virale Infektionen von Säugern, insbesondere des Menschen, sind weit verbreitet. Trotz intensiver Bemühungen ist es bisher nicht gelungen, Chemotherapeutika bereitzustellen, die ursächlich oder symptomatisch mit dem viral oder retroviral bedingten Krankheitsgeschehen mit erkennbar substantiellem Erfolg interferieren. Es ist heutzutage nicht möglich, bestimmte Viruserkrankungen, wie zum Beispiel das Acquired Immune Deficiency Syndrom (AIDS), den AIDS-related-complex (ARC) und deren Vorstadien, Herpes-, Cytomegalie-Virus (CMV)-, Influenza-und andere Virusinfektionen zu heilen oder chemotherapeutisch deren Symptome günstig zu beeinflussen. Derzeit steht beispielsweise für die Behandlung von AIDS fast ausschließlich das 3'-Azido-3'-deoxy-thymidin (AZT), bekannt als Zidovudine oder Retrovir$^{R}$, zur Verfügung. AZT ist jedoch durch eine sehr enge therapeutische Breite bzw. durch bereits im therapeutischen Bereich auftretende, sehr schwere Toxizitäten charakterisiert (Hirsch, M.S. (1988) J.Infec.Dis. 157, 427-431). Die Verbindungen der allgemeinen Formel I besitzen diese Nachteile nicht. Sie wirken antiviral, ohne in pharmakologisch relevanten Dosen cytotoxisch zu sein.

Es konnte nun nachgewiesen werden, daß Verbindungen der allgemeinen Formel I die Vermehrung von DNA- bzw. RNA-Viren auf der Stufe der virusspezifischen DNA- bzw. RNA-Transkription hemmen. Die Substanzen können über die Inhibierung des Enzyms Reverse Transkriptase die Vermehrung von Retroviren beeinflussen (vgl. Proc. Natl. Acad. Sci. USA 83, 1911, 1986 bzw. Nature 325, 773 1987).

Da ein sehr großer Bedarf an Chemotherapeutica besteht, die möglichst spezifisch mit retroviral bedingten Erkrankungen oder deren Symptomen interferieren, ohne die normal ablaufenden natürlichen Körperfunktionen zu beeinflussen, konnten die genannten Verbindungen vorteilhaft prophylaktisch oder therapeutisch bei der Behandlung von Krankheiten eingesetzt werden, bei denen eine retrovirale Infektion von pathophysiologischer, symptomatischer oder klinischer Relevanz ist.

Die Verbindungen der Formel I besitzen ein Chiralitätszentrum und können sowohl in Form ihrer Racemate als auch in Form ihrer Enantiomere oder Diastereoisomere eingesetzt werden. Die Trennung der Racemate in die Enantiomeren kann analytisch, semipräparativ und präparativ chromatographisch auf geeigneten optisch aktiven Phasen mit gängigen Elutionsmitteln durchgeführt werden. Als optisch aktive Phasen eignen sich beispielsweise optisch aktive Polyacrylamide oder Polymethacrylamide, z.T. auch an Kieselgel (z.B. ChiraSpher $^{(R)}$ von Merck, Chiralpak $^{(R)}$ OT/OP von Baker), Celluloseester/carbamate (z.B. Chiracel $^{(R)}$ OB/OY von Baker/Daicel), Phasen auf Cyclodextrin- oder Kronenetherbasis (z.B. Crownpak $^{(R)}$ von Daicel) oder mikrokristallines Cellulosetriacetat (Merck).

Ein aliphatischer Rest bedeutet einen geradkettigen oder verzweigten Alkyl-, Alkenyl- oder Alkinylrest mit 1-9, vorzugsweise 2-7 Kohlenstoffatomen, wie z.B. der Propyl-, Isopropyl-, Butyl-, Isobutyl-, Pentyl-, Hexyl- oder Heptylrest. Als ungesättigte Reste kommen $C_2$-$C_7$-Alkenyl- und Alkinylreste in Frage, bevorzugt $C_2$-$C_5$, wie z.B. der Allyl-, Dimethylallyl-, Butenyl-, Isobutenyl-, Pentenyl- oder Propinylrest.

Ein aliphatischer Rest, der durch Phenyl substituiert sein kann, ist insbesondere eine Phenyl-$C_1$-$C_6$-alkylgruppe, wie z.B. der Benzyl-, Phenethyl-, Phenylpropyl- oder Phenylbutylrest.

Bedeutet R einen Phenylring, so kann dieser ein-, zwei- oder dreifach substituiert sein. Die Substituenten können unabhängig voneinander in o-, m- oder p-Stellung stehen.

Ein carbocyclischer Ring mit 7-15 C-Atomen kann mono-, bi- oder tricyclisch sein und pro Ring jeweils 5 oder 6 C-Atome aufweisen. Dieser Ring kann gesättigt, ungesättigt, teilweise gesättigt oder aromatisch sein. Beispielhaft genannt seien die folgenden Ringsysteme: der Naphthyl-, Anthracenyl-, Phenanthrenyl-, Flourenyl-, Indenyl-, Indanyl-, Acenaphthylenyl-, Norbornyl-, Adamantylring oder eine $C_3$-$C_7$-Cycloalkyl- oder $C_5$-$C_8$-Cycloalkenylgruppe, wobei in den beiden letzten Fällen die entsprechende Fünf- oder Sechsringe bevorzugt sind.

Die heterocylischen mono-, bi- oder tricyclischen Ringsysteme enthalten pro Ringsystem 5 oder 6 Kohlenstoffatome, wobei 1-4 bzw. 1-5 C-Atome durch die Heteroatome Sauerstoff, Schwefel und/oder Stickstoff ersetzt sein können. Die Ringsysteme können aromatisch, partiell oder vollständig hydriert sein. Beispielhaft genannt seien die folgenden Ringsysteme: das Pyridin-, Pyrimidin-, Pyridazin-, Pyrazin- , Triazin-, Pyrrol-, Pyrazol-, Imidazol-, Triazol-, Thiazol-, Oxazol-, Isoxazol-, Oxadiazol-, Furazan-, Furan- , Thiophen-, Indol-, Chinolin-, Isochinolin-, Cumaron-, Thionaphthen-, Benzoxazol-, Benzthiazol-, Indazol-, Benzimidazol-, Benztriazol-, Chromen-, Phthalazin-, Chinazolin-, Chinoxalin-, Methylendioxybenzol-, Carbazol-, Acridin-, Phenoxazin-, Phenothiazin-, Phenazin- oder Purinsystem, wobei die ungesättigten bzw. aromatischen Carbo- und Heterocyclen partiell oder vollständig hydriert sein können.

Als Aryl- bzw. Hetarylreste kommen in der Definition von $R^3$-$R^6$ bevorzugt dar Phenyl-, Naphthyl- oder Pyridylrest in Frage, wobei diese Reste insbesondere ein- oder zweifach substituiert sein können durch $C_1$-$C_3$-Alkyl, $C_1$-$C_3$-Alkoxy oder Halogen.

R bedeutet bevorzugt unsubstituiertes Phenyl oder Phenyl ein- oder zweifach substituiert durch $C_1$-$C_3$-Alkyl, $C_1$-$C_3$-Alkoxy, $C_1$-$C_3$-Alkylmercapto, $C_1$-$C_3$-Alkylsulfinyl, $C_1$-$C_3$-Alkylsulfonyl, $C_2$-$C_4$-Alkenyl, $C_2$-$C_3$-Alkinyl, $C_3$-$C_4$-Alkenyloxy, $C_1$-$C_3$-Alkylamino, $C_1$-$C_3$-Dialkylamino-, $C_1$-$C_3$-Alkylcarbonylamino, $C_1$-$C_3$-Alkylaminocarbonyl, $C_1$-$C_3$-Alkoxycarbonyl-, Amino, Hydroxy, Nitro, Azido, Trifluormethyl, Cyano oder Halogen. Carbocylische Ringe sind bevorzugt Biphenyl, Naphthyl, Anthracenyl, Indanyl, Fluorenyl, Acenaphthylenyl, Phenanthrenyl, Norbornyl, Adamantyl, $C_3$-$C_6$-Cycloalkyl, $C_5$-$C_8$-Cycloalkenyl. Heterocyclische Ringsysteme sind bevorzugt Pyrrol, Imidazol, Furan, Thiophen, Pyridin, Pyrimidin, Thiazol, Triazin, Indol, Chinolin, Isochinolin, Cumaron, Thionaphthen, Benzimidazol, Chinazolin, Methylendioxybenzol, Ethylendioxybenzol, Carbazol, Acridin und Phenothiazin.

Für die Reste $R^1$ und $R^2$ sind Wasserstoff, $C_1$-$C_3$-Alkyl, $C_2$-$C_4$-Alkenyl, $C_2$-$C_4$-Alkinyl, $C_1$-$C_3$-Alkoxy, $C_1$-$C_3$-Alkylmercapto, $C_1$-$C_3$-Alkylamino, $C_1$-$C_3$-Alkoxycarbonyl, Sulfonamido, Amino, Halogen, Hydroxy, Cyano und Azido bevorzugt, wobei diese Reste insbesondere in 7-, 8- oder 9-Stellung des Thiazolo[2,3-a]-isoindolringes stehen.

Bevorzugte Substituenten für $R^3$, $R^4$, $R^5$ und $R^6$ sind Wasserstoff, $C_1$-$C_3$-Alkyl, $C_1$-$C_3$-Alkoxy, $C_1$-$C_3$-Alkylmercapto, Carboxy, $C_1$-$C_3$-Alkoxycarbonyl, Morpholinocarbonyl, Aminocarbonyl, $C_1$-$C_3$-Alkylaminocarbonyl, Di-$C_1$-$C_3$-alkylaminocarbonyl, $C_1$-$C_3$-Alkoxy-$C_1$-$C_3$-alkoxycarbonyl, Pyridyl-$C_1$-$C_3$-alkoxycarbonyl, Halogen, Cyano und Hydroxy, wobei $R^3$ und $R^4$ insbesondere Wasserstoff bedeutet. Die Reste $R^3$-$R^6$ können gleich oder verschieden sein, bevorzugt sind jedoch solche Derivate, in denen mindestens zwei, bevorzugt drei dieser Reste Wasserstoff bedeuten.

X ist bevorzugt Sauerstoff oder Schwefel, n bevorzugt gleich 0. Unter Halogen ist allgemein Fluor, Chlor, Brom und Iod zu verstehen, bevorzugt Fluor, Chlor und Brom.

Besonders bevorzugte Reste für R sind $C_3$-$C_5$-Alkyl, $C_2$-$C_5$-Alkenyl, $C_2$-$C_4$-Alkinyl, Benzyl, Phenethyl; Phenyl; durch $C_1$-$C_3$-Alkyl, $C_1$-$C_3$-Alkoxy, $C_1$-$C_3$-Alkylmercapto, Allyl, Allyloxy, $C_1$-$C_3$-Alkylamino, Di-$C_1$-$C_3$-alkylamino, Amino, Hydroxy, Azido, Nitro, Trifluormethyl, Cyano oder Halogen mono- oder disubstituiertes Phenyl bzw. durch Methyl oder Halogen trisubstituiertes Phenyl; Naphthyl, Anthracenyl, Indanyl, Acenaphthylenyl, Phenanthrenyl, Adamantyl, Cyclohexyl, Cyclohexenyl, Furyl, Thienyl, Pyridyl, Pyrimidinyl, Thiazolyl, Indolyl, Chinolinyl, Benzimidazolyl, Methyiendioxyphenyl, Carbazolyl und Phenothiazinyl.

Für $R^1$ und $R^2$ sind unabhängig voneinander besonders bevorzugt Wasserstoff, Methyl, Ethyl, Isopropyl, Allyl, Methoxy, Ethoxy, Methylmercapto, Ethylmercapto, Methylamino, Methoxycarbonyl, Ethoxycarbonyl, Amino, Azido, Cyano, Hydroxy und Halogen, wobei Halogen insbesondere Chlor und Brom bedeutet.

Für $R^3$, $R^4$, $R^5$ und $R^6$ sind Methyl, Ethyl, Isopropyl, Methoxy, Ethoxy, Methylmercapto, Ethylmercapto, Methylamino, Amino, Chlor, Brom und Cyano besonders bevorzugt.

Insbesondere bevorzugt sind Verbindungen der allgemeinen Formel I, in denen R, $R^1$, X und n die oben angegebene Bedeutung haben und $R^2$, $R^3$, $R^4$, $R^5$ und $R^6$ gleich Wasserstoff, Methyl, Ethyl, Chlor, Brom, Methoxy oder Ethoxy sind, wobei die Reste $R^2$ bis $R^6$ bevorzugt Wasserstoff darstellen.

Die neuen Verbindungen der Formel I, in der R einen heterocylischen Rest bedeutet, sind insbesondere solche Derivate, in denen R Thienyl, Furyl, Pyridyl, Thionaphthenyl oder Indolyl bedeutet, wobei diese Reste insbesondere durch $C_1$-$C_6$-Alkyl und Halogen substituiert sein können.

Die Arzneimittel enthaltend mindestens eine Verbindung der Formel I zur Behandlung von viralen Infektionen können in flüssiger oder fester Form enteral oder parenteral appliziert werden. Hierbei kommen die üblichen Applikationsformen in Frage, wie beispielsweise Tabletten, Kapseln, Dragées, Sirupe, Lösungen oder Suspensionen. Als Injektionsmedium kommt vorzugsweise Wasser zur Anwendung, das die bei Injektionslösungen üblichen Zusätze wie Stabilisierungsmittel, Lösungsvermittler und Puffer enthält. Derartige Zusätze sind z.B. Tartrat- und Zitratpuffer, Ethanol, Komplexbildner, wie Ethylen-diamintetraessigsäure und deren nichttoxischen Salze, hochmolekulare Polymere, wie flüssiges Polyethylenoxid zur Viskositätsregulierung. Flüssige Trägerstoffe für Injektionslösungen müssen steril sein und werden vorzugsweise in Ampullen abgefüllt. Feste Trägerstoffe sind beispielsweise Stärke, Lactose, Mannit, Methylcellulose, Talkum, hochdisperse Kieselsäuren, höher molekulare Fettsäuren, wie Stearinsäure, Gelatine, Agar-Agar, Calziumphosphat, Magnesiumstearat, tierische und pflanzliche Fette, feste hochmolekulare Polymere, wie Polyethylenglykole, etc.. Für orale Applikationen geeignete Zubereitungen können gewünschtenfalls Geschmacks- oder Süßstoffe enthalten.

Die Arzneimittel enthaltend mindestens eine Verbindung der Formel I werden dadurch hergestellt, indem man eine Verbindung der Formel I mit üblichen pharmazeutischen Hilfsstoffen mischt und zu Arzneiformen, wie z. B. Tabletten, Dragees, Kapseln oder Lösungen verarbeitet. Diese Arzneiformen werden zu verkaufsfertigen Verpackungseinheiten konfektioniert, und mit einem entsprechenden Hinweis, z. B. in Form eines Beipackzettels, versehen, aus dem die Verwendung zur Behandlung von viralen oder retroviralen Infektionen bzw. von viralen oder retroviral bedingten Erkrankungen hervorgeht.

Die Dosierung kann von verschiedenen Faktoren, wie Applikationsweise, Spezies, Alter oder individuellem Zustand abhängen. Die erfindungsgemäßen Verbindungen werden üblicherweise in Mengen von 0,1 - 100 mg, vorzugsweise 0,2 - 80 mg pro Tag und pro kg Körpergewicht appliziert. Bevorzugt ist es, die Tagesdosis auf 2-5 Applikationen zu verteilen, wobei bei jeder Applikation 1-2 Tabletten mit einem Wirkstoffgehalt von 0,5 - 500 mg verabreicht werden. Die Tabletten können auch retardiert sein, wodurch sich die Anzahl der Applikationen pro Tag auf 1-3 vermindert. Der Wirkstoffgehalt der retardierten Tabletten kann 2 - 1000 mg betragen. Der Wirkstoff kann auch durch Dauerinfusion gegeben werden, wobei die Mengen von 5 - 1000 mg pro Tag normalerweise ausreichen.

Die Verbindungen der Formel I können auch in Form ihrer physiologisch verträglichen Salze, wie z.B. der Alkali- oder Erdalkalisalze eingesetzt werden, sofern diese Verbindungen saure Gruppen, wie z.B. eine freie Carboxygruppe besitzen. Sind basische Gruppen vorhanden, so können die Verbindungen der Formel I mit Hilfe von organischen oder anorganischen Säuren in die entsprechenden physiologisch verträgliche Säureadditionssalze überführt werden.

Die erfindungsgemäßen Verbindungen der allgemeinen Formel I werden nach literaturbekannten Verfahren hergestellt, indem man gegebenenfalls substituierte Benzoesäurederivaten der allgemeinen Formel II

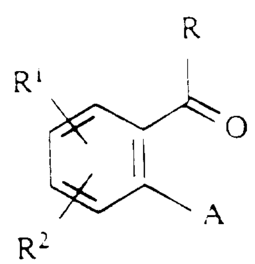

(II),

in der R, $R^1$, und $R^2$ die oben angegebene Bedeutung haben und A gleich -COOH oder C=N ist, mit substituiertem oder unsubstituiertem Cysteamin der allgemeinen Formel III

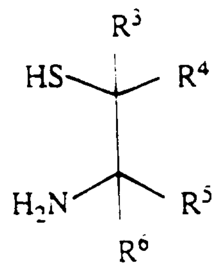

(III),

in der $R^3$, $R^4$, $R^5$ und $R^6$ die oben angegebene Bedeutung haben, in einem geeigneten inerten Lösungsmittel bei Raumtemperatur bis Rückflußtemperatur evtl. in Gegenwart katalytischer Mengen Säure, z.B. p-Toluolsulfonsäure, umsetzt und gegebenenfalls anschließend Verbindungen der Formel I in andere Verbindungen der Formel I nachträglich umwandelt und anschließend chromatographisch bzw. durch Umkristallisation reinigt. Racemate können durch Chromatographie an geeigneten optisch aktiven Phasen, z.B. Cellulosetriacetat, in die Antipoden getrennt werden.

Die nachträglichen Umwandlungen von Verbindungen der Formel I in andere Verbindungen der Formel I betreffen die Herstellung von Thialzolo-[2,3-a]isoindolin-Derivaten mit X = S oder N-Alkylimin. Verbindungen mit X = S werden hergestellt durch Umsetzung von Verbindungen der Formel I, in der X ein Sauerstoffatom bedeutet, mit schwefelgruppenübertragenden Verbindungen, wie z.B. Lawesson's Reagenz. Verbindungen mit X = N-Alkylimino werden hergestellt durch Umsetzung der entsprechenden Iminoverbindungen der allgemeinen Formel I mit Alkylaminen nach an sich bekannten Methoden.

Die Benzoesäurederivate der allgemeinen Formel II sind ebenfalls literaturbekannt und werden z.B. durch Friedel-Crafts-Acylierung von substituiertem oder unsubstituiertem Phthalsäureanhydrid mit gegebenenfalls substituierten Arenen in Gegenwart einer Lewis-Säure (z.B. Aluminiumchlorid) oder durch Reaktion von Grignardreagenzien der allgemeinen Formel IV

R-MgBr (IV),

in der R mit Ausnahme von Wasserstoff die oben angegebene Bedeutung hat, mit Phthalsäureanhydrid, das gegebenenfalls substituiert ist, in geeigneten inerten Lösungsmitteln bei tiefen Temperaturen hergestellt.

Die Verfahren zur Herstellung der erfindungsgemäßen Verbindungen der allgemeinen Formel I können auch der im Stand der Technik genannten Patentanmeldungen bzw. Literaturstellen entnommen werden (vgl. US-Patent 3,334,113, CH-469,733, belgische Patentanmeldung 659,528 bzw. US-Patent 3,646,022, U.S. 2,860,985, belgische Patentanmeldung 564,592, J. Org. Chem. 30, 1506 (1965) sowie J. Org. Chem. 34, 165 (1969) ).

Im Sinne der vorliegenden Erfindung kommen außer den in den Beispielen genannten Verbindungen und der durch Kombination aller in den Ansprüchen genannten Bedeutungen der Substituenten die folgenden Verbindungen der Formel I in Frage, die als racemischen Gemische oder in optisch aktiver Form bzw. als reine R- und S-Enantiomeren vorliegen können:

1. 8,9b-Dimethyl-2,3-dihydrothiazolo-[2,3-a]isoindol-5(9bH)-on
2. 8-Chlor-9b-phenyl-2,3-dihydrothiazolo-[2,3-a]isoindol-5(9bH)-on
3. 8-Fluor-9b-(4-methylphenyl)-2,3-dihydrothiazolo-[2,3-a]isoindol-5(9bH)-on
4. 8-Chlor-9b-(3-methylphenyl)-2,3-dihydrothiazolo-[2,3-a]isoindol-5(9bH)-on
5. 3-Methyl-9b-(4-ethylphenyl)-2,3-dihydrothiazolo-[2,3-a]isoindol-5(9bH)-on
6. 9b-(2,3-Dimethylphenyl)-2,3-dihydrothiazolo-[2,3-a]isoindol-5(9bH)-thion
7. 8-Chlor-9b-(3,4-dimethylphenyl)-2,3-dihydrothiazolo-[2,3-a]isoindol-5(9bH)-thion
8. 2-Ethyl-9b-(2,5-Dimethylphenyl)-2,3-dihydrothiazolo-[2,3-a]isoindol-5(9bH)-on
9. 8-Chlor-9b-(3-trifluormethylphenyl)-2,3-dihydrothiazolo-[2,3-a]isoindol-5(9bH)-on
10. 6-Methoxy-9b-(4-trifluormethylphenyl)-2,3-dihydrothiazolo-[2,3-a]isoindol-5(9bH)-on
11. 9b-(4-Hydroxyphenyl)-2,3-dihydrothiazolo-[2,3-a]isoindol-5(9bH)-thion
12. 8-Chlor-9b-(3-hydroxyphenyl)-2,3-dihydrothiazolo-[2,3-a]isoindol-5(9bH)-on
13. 7-Methylmercapto-9b-(4-ethoxyphenyl)-2,3-dihydrothiazolo-[2,3-a]isoindol-5(9bH)-on
14. 9-Methyl-9b-(3-methoxyphenyl)-2,3-dihydrothiazolo-[2,3-a]isoindol-5(9bH)-on
15. 8-Fluor-9b-(3-fluorphenyl)-2,3-dihydrothiazolo-[2,3-a]isoindol-5(9bH)-on
16. 9b-(4-Chlorphenyl)-2,3-dihydrothiazolo-[2,3-a]isoindol-5(9bH)-thion
17. 8-Methyl-9b-(3-methylsulfonylphenyl)-2,3-dihydrothiazolo-[2,3-a]isoindol-5(9bH)-on

18. 9-Chlor-9b-phenyl-2,3-dihydrothiazolo-[2,3-a]isoindol-5(9bH)-on-1-oxid
19. 8-Chlor-9b-benzyl-2,3-dihydrothiazolo-[2,3-a]isoindol-5(9bH)-on
20. 2,2,-Dimethyl-9b-phenethyl-2,3-dihydrothiazolo-[2,3-a]isoindol-5(9bH)-on
21. 9b-(3-Methylmercaptophenyl)-2,3-dihydrothiazolo-[2,3-a]isoindol-5(9bH)-on
22. 9b-(3-Methylaminophenyl)-2,3-dihydrothiazolo-[2,3-a]isoindol-5(9bH)-on
23. 9b-(3-Azidophenyl)-2,3-dihydrothiazolo-[2,3-a]isoindol-5(9bH)-on
24. 8-Methyl-9b-allyl-2,3-dihydrothiazolo-[2,3-a]isoindol-5(9bH)-on
25. 8-Chlor-9b-(3,5-dimethylphenyl)-2,3-dihydrothiazolo-[2,3-a]isoindol-5(9bH)-on
26. 8-Methyl-9b-(1-napthyl)-2,3-dihydrothiazolo-[2,3-a]isoindol-5(9bH)-on
27. 9b-(Anthracen-1-yl)-2,3-dihydrothiazolo-[2,3-a]isoindol-5(9bH)-thion
28. 9b-(Anthracen-9-yl)-2,3-dihydrothiazolo-[2,3-a]isoindol-5(9bH)-on
29. 9b-(Inden-1-yl)-2,3-dihydrothiazolo-[2,3-a]isoindol-5(9bH)-on
30. 9b-(Inden-3-yl)-2,3-dihydrothiazolo-[2,3-a]isoindol-5(9bH)-on
31. 9b-(Inden-4-yl)-2,3-dihydrothiazolo-[2,3-a]isoindol-5(9bH)-thion
32. 9b-(Phenanthren-1-yl)-2,3-dihydrothiazolo-[2,3-a]isoindol-5(9bH)-on
33. 9b-(Phenanthren-9-yl)-2,3-dihydrothiazolo-[2,3-a]isoindol-5(9bH)-on
34. 9b-(Cyclohexen-3-yl)-2,3-dihydrothiazolo-[2,3-a]isoindol-5(9bH)-thion
35. 9b-(2-Furyl)-2,3-dihydrothiazolo-[2,3-a]isoindol-5(9bH)-thion
36. 9b-(3-Furyl)-2,3-dihydrothiazolo-[2,3-a]isoindol-5(9bH)-on
37. 9b-(2-Thienyl)-2,3-dihydrothiazolo-[2,3-a]isoindol-5(9bH)-thion
38. 9b-(3-Thienyl)-2,3-dihydrothiazolo-[2,3-a]isoindol-5(9bH)-on
39. 9b-(Pyrimidin-4-yl)-2,3-dihydrothiazolo-[2,3-a]isoindol-5(9bH)-on
40. 9b-(Thiazol-2-yl)-2,3-dihydrothiazolo-[2,3-a]isoindol-5(9bH)-on
41. 9b-(Thiazol-4-yl)-2,3-dihydrothiazolo-[2,3-a]isoindol-5(9bH)-thion
42. 9b-(Indol-3-yl)-2,3-dihydrothiazolo-[2,3-a]isoindol-5(9bH)-on
43. 9b-(Indol-7-yl)-2,3-dihydrothiazolo-[2,3-a]isoindol-5(9bH)-on
44. 9b-(Chinolin-4-yl)-2,3-dihydrothiazolo-[2,3-a]isoindol-5(9bH)-on
45. 9b-(Chinolin-5-yl)-2,3-dihydrothiazolo-[2,3-a]isoindol-5(9bH)-thion
46. 9b-(Benzimidazol-4-yl)-2,3-dihydrothiazolo-[2,3-a]isoindol-5(9bH)-on
47. 9b-(Carbazol-1-yl)-2,3-dihydrothiazolo-[2,3-a]isoindol-5(9bH)-on
48. 9b-(Carbazol-4-yl)-2,3-dihydrothiazolo-[2,3-a]isoindol-5(9bH)-thion
49. 9b-(Phenothiazin-1-yl)-2,3-dihydrothiazolo-[2,3-a]isoindol-5(9bH)-thion
50. 9b-(Phenothiazin-4-yl)-2,3-dihydrothiazolo-[2,3-a]isoindol-5(9bH)-on
51. 9b-(4-Chinazolin-4-yl)-2,3-dihydrothiazolo-[2,3-a]isoindol-5(9bH)-on
52. 8-Chlor-9b-(inden-3-yl)-2,3-dihydrothiazolo-[2,3-a]isoindol-5(9bH)-on
53. 8-Methyl-9b-(isochinolin-1-yl)-2,3-dihydrothiazolo-[2,3-a]isoindol-5(9bH)-thion
54. 9-Methoxy-9b-(1-naphthyl)-2,3-dihydrothiazolo-[2,3-a]isoindol-5(9bH)-on
55. 9b-(Cumaron-3-yl)-2,3-dihydrothiazolo-[2,3-a]isoindol-5(9bH)-on
56. 9b-(1-Naphthyl)-2,3-dihydrothiazolo-[2,3-a]isoindol-5(9bH)-on-1,1-dioxid
57. 9b-(1-Naphthyl)-2,3-dihydrothiazolo-[2,3-a]isoindol-5(9bH)-on-1-oxid

Beispiel 1

9b-(1-Naphthyl)-2,3-dihydrothiazolo-[2,3-a]isoindol-5(9bH)-on

2.76 g (10 mmol) 2-(1-Naphthoyl)benzoesäure wurden in 100 ml Xylol gelöst und nach Zugabe von 1.54 g (20 mmol) Cysteamin sowie einer katalytischen Menge p-Toluolsulfonsäure 1 h am Wasserabscheider unter Rückfluß erhitzt. Dann wurde das Lösungsmittel im Vakuum entfernt und der Rückstand aus Ethanol umkristallisiert. Ausbeute 1.54 g (67 % d.Th.), Schmp. 151-152 °C.

Die verwendete 2-(1-Naphthoyl)benzoesäure wurde durch langsames Zutropfen von 1-Napthylmagnesiumbromid in Ether/Toluol 4/1 bei -10 °C zu einer Lösung von Phthalsäureanhydrid in Toluol, nach 2-stündigem Nachrühren Zugabe von ges. $NH_4Cl$-Lösung, Extraktion mit Essigester, Ausschütteln der Essigesterphase mit 2 N Sodalösung und erneuter Extraktion der angesäuerten Sodaphase mit Essigester hergestellt. Ausbeute nach Umkristallisation aus Ethanol 64 % d.Th., Schmp. 170 °C.

Analog zu Bsp. 1 wurden folgende Verbindungen hergestellt:

1.1 9b-(2-Naphthyl)-2,3-dihydrothiazolo-[2,3-a]isoindol-5(9bH)-on; amorph; Rf = 0.5 (Essigester/Isohexan 1/3) aus 2-(2-Naphthoyl)benzoesäure und Cysteamin (66 % Ausb.)

1.2 9b-(Anthracen-9-yl)-2,3-dihydrothiazolo-[2,3-a]isoindol-5(9bH)-on; Schmp. 198 °C aus 2-(9-Anthracenoyl)-benzoesäure und Cysteamin ( 49 % Ausb.)

1.3 7-Chlor-9b-phenyl-2,3-dihydrothiazolo-[2,3-a]isoindol-5(9bH)-on; Schmp. 123 °C aus 5-Chlor-2-benzoylbenzoesäure und Cysteamin (61 % Ausb.)

1.4 7-Methyl-9b-phenyl-2,3-dihydrothiazolo-[2,3-a]isoindol-5(9bH)-on; Schmp. 98 °C aus 5-Methyl-2-benzoylbenzoesäure und Cysteamin (59 % Ausb.)

1.5 6-Methyl-9b-phenyl-2,3-dihydrothiazolo-[2,3-a]isoindol-5(9bH)-on; Schmp. 185 °C aus 6-Methyl-2-benzoylbenzoesäure und Cysteamin (79 % Ausb.)

1.6 7-Methoxy-9b-phenyl-2,3-dihydrothiazolo-[2,3-a]isoindol-5(9bH)-on; $R_f$ = 0.33; (Essigester/Isohexan 1/3) aus 5-Methoxy-2-benzoylbenzoesäure und Cysteamin (55 % Ausb.)

1.7 7,8-Dichlor-9b-phenyl-2,3-dihydrothiazolo-[2,3-a]isoindol-5(9bH)-on; Schmp. 112-114 °C aus 4,5-Dichlor-2-benzoylbenzoesäure und Cysteamin (67 % Ausb.)

1.8 9b-(2-Thienyl)-2,3-dihydrothiazolo[2,3-a]isoindol-5(9bH)-on; Schmp. 151 °C (Ethanol/$H_2O$) aus 2-(2-Thienoyl)benzoesäure und Cysteamin (63 % Ausb.)

1.9 9b-(2-Furyl)-2,3-dihydrothiazolo[2,3-a]isoindol-5(9bH)-on; Schmp. 114 °C (Ethanol/Ether) aus 2-(2-Furoyl)-benzoesäure und Cysteamin (70 % Ausb.)

1.10 9b-Cyclopentyl-2,3-dihydrothiazolo-[2,3-a]isoindol-5(9bH)-on; Öl; $R_f$ = 0.85 ($CH_2Cl_2$/$CH_3OH$ 9/1) aus 2-Cyclopentoylbenzoesäure und Cysteamin (81 % Ausb.). Die Reinigung dieser Verbindung erfolgt durch Säulenchromatographie mit Essigester/Isohexan 1/1.

1.11 8-Chlor-7-sulfonamido-9b-phenyl-2,3-dihydrothiazolo-[2,3-a]isoindol-5-(9bH)-on; Schmp. 245-246 °C aus 4-Chlor-5-sulfonamido-2-benzoylbenzoesäure und Cysteamin (54 % Ausb.)

1.12 8-Chlor-9b-phenyl-2,3-dihydrothiazolo[2,3-a]isoindol-5(9bH)-on; Schmp. 113-136 °C aus 4-Chlor-2-benzoylbenzoesäure und Cysteamin (68 % Ausb.)

1.13 8-Methyl-9b-phenyl-2,3-dihydrothiazolo-[2,3-a]isoindol-5(9bH)-on; Schmp. 115-118 °C aus 4-Methyl-2-benzoylbenzoesäure und Cysteamin (75 % Ausb.)

1.14 9b-(4-Pyridyl)-2,3-dihydrothiazolo[2,3-a]isoindol-5(9bH)-on;Schmp. 114 °C aus 2-(4-Pyridoyl)-benzoesäure und Cysteamin (68 % Ausb.)

1.15 9b-(2-Pyridyl)-2,3-dihydrothiazolo[2,3-a]isoindol-5(9bH)-on;Schmp. 115-116 °C aus 2-(2-Pyridoyl)-benzoesäure und Cysteamin (61 % Ausb.)

1.16 9b-(3-Pyridyl)-2,3-dihydrothiazolo[2,3-a]isoindol-5(9bH)-on;Schmp. 149-152 °C aus 2-(3-Pyridoyl)-benzoesäure und Cysteamin (57 % Ausb.)

1.17 9b-Cyclohexyl-2,3-dihydrothiazolo[2,3-a]isoindol-5(9bH)-on; Öl; $R_f$ = 0.55 (Essigester/Isohexan 1/3) aus 2-Cyclohexoylbenzoesäure und Cysteamin (79 % Ausb.)

1.18 9b-(2-Aminophenyl)-2,3-dihydrothiazolo-[2,3-a]isoindol-5(9bH)-on; Schmp. 147-151 °C (Isopropanol) aus 2-(2-Aminobenzoyl)benzoesaure und Cysteamin (43 % Ausb.)

1.19 9b-(4-Aminophenyl)-2,3-dihydrothiazolo-[2,3-a]isoindol-5(9bH)-on; Schmp. 179-185 °C (Isopropanol) aus 2-(4-Aminobenzoyl)benzoesäure und Cysteamin (49 % Ausb.).

1.20 9b-(Indan-4-yl)-2,3-dihydrothiazolo-[2,3-a]isoindol-5(9bH)-on; Schmp. 151-153 °C aus 2-(Indan-4-ylcarbonyl)-benzoesäure und Cysteamin (63 % Ausb.)

1.21 9b-(2-Nitro-5-methylphenyl)-2,3-dihydrothiazolo-[2,3-a]isoindol-5(9bH)-on; Schmp. 161-164 °C (Essigester/Isohexan) aus 2-(2-Nitro-5-methyl-benzoyl)benzoesäure und Cysteamin (70 % Ausb.)

1.22 8-Methoxy-9b-phenyl-2,3-dihydrothiazolo-[2,3-a]isoindol-5(9bH)-on; Öl aus 4-Methoxybenzoylbenzoesäure und Cysteamin (56 % Ausb.)

1.23 9b-(3-Nitrophenyl)-2,3-dihydrothiazolo-[2,3-a]isoindol-5(9bH)-on; Schmp. 91-97 °C aus 2-(3-Nitrobenzoyl)benzoesäure und Cysteamin (33 % Ausb.)

1.24 8-Chlor-9b-(1-naphthyl)-2,3-dihydrothiazolo-[2,3-]isoindol-5(9bH)-on; Schmp. 156-159 °C (Methanol) aus 4-Chlor-2-(1-naphthoyl)benzoesäure und Cysteamin (28 % Ausb.)

1.25 9b-(3-Dimethylaminophenyl)-2,3-dihydrothiazolo-[2,3-a]-isoindol-5(9bH)-on; Schmp. 149-151 °C (Methanol) aus 2-(3-Dimethylaminobenzoyl)benzoesäure und Cysteamin (27 % Ausb.)

1.26 9b-(9-Phenanthrenyl)-2,3-dihydrothiazolo-[2,3-a]isoindol-5(9bH)-on; Schmp. 170-172 °C (Essigester/Isohexan) aus 2-(9-Phenanthrenoyl)benzoesäure und Cysteamin (64 % Ausb.)

1.27 9b-(3-Amino-4-chlorphenyl)-2,3-dihydrothiazolo-[2,3-a]isoindol-5(9bH)-on; Schmp. 180 °C (Isopropanol) aus 2-(3-Amino-4-chlorbenzoyl)benzoesäure und Cysteamin (41 % Ausb.)

1.28 9b-(5-Fluor-1-naphthyl)-2,3-dihydrothiazolo-[2,3-a]isoindol-5(9bH)-on; Schmp. 157 °C aus 2-(5-Fluor-1-naphthoyl)benzoesäure und Cysteamin (85 % Ausb.)

1.29 8-Chlor-9b-(3-Chlorphenyl)-2,3-dihydrothiazolo-[2,3-a]isoindol-5(9bH)-on; Schmp. 162 °C aus 4-Chlor-2-(3-chlorbenzoyl)benzoesäure und Cysteamin (73 % Ausb.)

1.30 6-Methoxy-9b-phenyl-2,3-dihydrothiazolo-[2,3-a]isoindol-5(9bH)-on; Schmp. 187 °C (Isopropanol) aus 6-Methoxy-2-benzoylbenzoesäure und Cysteamin (42 % Ausb.)

1.31 8-Chlor-9b-(3-methylphenyl)-2,3-dihydrothiazolo-[2,3-a]isoindol-5(9bH)-on; Schmp. 129-132 °C (Ether) aus 4-Chlor-2-(3-methylbenzoyl)benzoesäure und Cysteamin (24 % Ausb.)

1.32 7-Chlor-9b-(3-methylphenyl)-2,3-dihydrothiazolo-[2,3-a]isoindol-5(9bH)-on; Schmp. 76-80 °C (Ether) aus 5- Chlor-2-(3-methylbenzoyl)benzoesäure und Cysteamin (27 % Ausb.)

1.33 9b-(4-Methylpyridin-2-yl)-2,3-dihydrothiazolo-[2,3-a]isoindol-5(9bH)-on; Öl aus 2-[2-(4-Methylpyridoyl)]benzoesäure und Cysteamin (53 % Ausb.)

1.34 9b-(2-Thionapthenyl)-2,3-dihydrothiazolo-[2,3-a]isoindol-5(9bH)-on; Schmp. 130-133 °C aus 2-(2-Thionaphthenoyl)benzoesäure und Cysteamin (62 % Ausb.)

1.35 9b-(3-Thionaphthenyl)-2,3-dihydrothiazolo-[2,3-a]isoindol-5(9bH)-on; Schmp. 206-217 °C aus 2-(3-Thionaphthenoyl)benzoesäure und Cysteamin (50 % Ausb.)

1.36 9b-(Indol-3-yl)-2,3-dihydrothiazolo-[2,3-a]isoindol-5(9bH)-on; Schmp. 272-275 °C (Methanol) aus 2-(Indol-3-ylcarbonyl)benzoesäure und Cysteamin (42 % Ausb.)

Die Verbindungen wurden jeweils aus Ethanol umkristallisiert, sofern nichts anderes angegeben ist.

Beispiel 2

9b-Phenyl-2,3-dihydrothiazolo-[2,3-a]isoindol-5(9bH)-thion

2 g (7.5 mmol) 9b-Phenyl-2,3-dihydrothiazolo-[2,3-a]isoindolin-5(9bH)-on [J.Org.Chem. 34, 165 (1969)] in 100 ml abs. Dioxan wurden mit 3.8 g (9.4 mmol) Lawesson's-Reagenz [2,4-Bis-(4-methoxyphenyl)-1,3-dithia-2,4-diphosphetan-2,4-disulfid] versetzt und 5 h bei 60 °C gerührt (DC-Kontrolle).

Nach dem Abkühlen wurde von Niederschlag abfiltriert, das Filtrat im Vakuum eingedampft und der Rückstand durch Flash-Säulenchromatographiemit Heptan/Methylethylketon 6/1 als Eluens gereinigt. Ausbeute 1.24 g (58 % d.Th.), Schmp. 152-155 °C.

Analog wurde hergestellt:

2.1 9b-(1-Naphthyl)-2,3-dihydrothiazolo-[2,3-a]isoindol-5(9bH)-thion
wurde aus der entsprechenden Oxo-Verbindung (Bsp. 1) hergestellt. Ausbeute: 71 % d.Th., Schm. °C.

Beispiel 3

Enantiomerentrennung von rac-9b-(1-Naphthyl)-2,3-dihydrothiazolo-[2,3-a]isoindol-5(9bH)-on an Cellulosetriacetat

Für die Trennung der Antipoden wurden 200 mg des Racemats in 15 ml Methanol gelöst, auf eine Säule mit 50 mm Innendurchmesser und 300 mm Länge (entsprechend 250 g Cellulosetriacetat, 15-25 µ Korngröße, Merck 16326) aufgegeben und mit Methanol eluiert (Fluß 7.5 ml/min, ca. 1.5 bar).

| | | Peak I | Peak II |
|---|---|---|---|
| UV-Detektion | [nm] : | 254 | 254 |
| Run Time | [min]: | 110 | 255 |
| $[\alpha]^{20}_{D}$ * | : | -454° +/-5 | +454° +/-5 |
| Schmp. | [°C] : | 175-176 | 175-176 |
| abs. Konfiguration | | (S) | (R) |

Die Enantiomeren wurden aus Methanol umkristallisiert.

* Enantiomerenreinheit nach HPLC jeweils >99.6 % ee

Analog zu Bsp. 3 wurden getrennt:

3.1 (-)-8-Chlor-9b-phenyl-2,3-dihydrothiazolo-[2,3-a]isoindol-5(9bH)-on (Schmp. 87-93 °C).

3.2 (+)-8-Chlor-9b-phenyl-2,3-dihydrothiazolo-[2,3-a]isoindol-5(9bH)-on (Schmp. 87-93 °C); mit Ethanol als Eluens.

3.3 (-)-8-Chlor-9b-(3-chlorphenyl)-2,3-dihydrothiazolo-[2,3-a]isoindol-5(9bH)-on; (Schmp. 138 °C/Ethanol; $[\alpha]_D$ = -236 °C/c = 1/$CHCl_3$)

3.4 (+)-8-Chlor-9b-(3-chlorphenyl)-2,3-dihydrothiazolo-[2,3-a]isoindol-5(9bH)-on; (Schmp. 138 °C/Etnanol; $[\alpha]_D$ = + 236 °C/c = 1/$CHCl_3$) mit Methanol als Eluens.

## Beispiel 4

### 9b-(4-Azidophenyl)-2,3-dihydrothiazolo-[2,3-a]isoindol-5(9bH)-on

2.1 g (7.4 mmol) 9b-(4-Aminophenyl)-2,3-dihydrothiazolo-[2,3-a]-isoindol-5(9bH)-on wurden in 12 ml 2 N HCl suspendiert, bei 0-5 °C innerhalb von 15 min. mit einer Lösung aus 0.55 g (8 mmol) $NaNO_2$ in 3 ml $H_2O$ versetzt und 30 min. bei 0 °C gerührt.

Dann wurde innerhalb von 10 min eine Lösung aus 0.6 g (9.2 mmol) $NaN_3$ in 8 ml $H_2O$ zugetropft, 30 min nachgerührt und der entstandene Niederschlag abgesaugt. Das Rohprodukt wurde durch Säulenchromatographie an Kieselgel 60 mit Ether/Isohexan 1/2 als Eluens gereinigt. Ausbeute 1.62 g (71 % d.Th.), Schmp. >140 °C Zers..

4.1 9b-(3-Azidophenyl)-2,3-dihydrothiazolo-[2,3-a]isoindol-5(9bH)-on wurde analog zu Bsp. 4 aus 9b-(3-Aminophenyl)-2,3-dihydrothiazolo-[2,3-a]isoindol-5(9bH)-on in 86 % Ausbeute hergestellt. Schmp. 100-101 °C (Umkristallisation aus Ether).

## Beispiel 5

### 9b-(3-Aminophenyl)-2,3-dihydrothiazolo-[2,3-a]isoindol-5(9bH)-on

11.2 g (35.7 mmol) 9b-(3-Nitrophenyl)-2,3-dihydrothiazolo-[2,3-a]isoindol-5(9bH)-on in 90 ml Ethanol wurden in der Siedehitze innerhalb von 5 min mit 23 g $Na_2S_2O_4$ in 90 ml $H_2O$ versetzt und 1 h unter Rückfluß erhitzt.

Dann wurde das Ethanol im Vakuum abgedampft, die wäßrige Phase mehrmals mit Dichlormethan extrahiert und die organische Phase über $Na_2SO_4$ getrocknet. Nach Entfernen des Lösungsmittels im Rotationsverdampfer wurde der Rückstand aus Dichlormethan umkristallisiert. Aus.b. 6.02 g (60 % d.Th.), Schmp. 184-186 °C.

5.1 9b-(2-Amino-5-methylphenyl)-2,3-dihydrothiazolo-[2,3-a]isoindol-5(9bH)-on
wurde analog zu Bsp. 5 aus 9b-(2-Nitro-5-methylphenyl)-2,3-dihydrothiazolo-[2,3-a]isoindol-5(9bH)-on in 53 % Ausbeute hergestellt. Schmp. 153-156 °C (Umkristallisation aus Dichlormethan).

Beispiel 6

9b-Phenyl-2,3-dihydrothiazolo-[2,3-a]isoindol-5(9bH)-on-3-carbonsäuremethylester

In eine Lösung aus 1 g (4.4 mmol) 2-Benzoylbenzoesäure in 10 ml Xylol wurden bei 100 °C über einen Zeitraum von 10 h portionsweise 1.7 g (9.9 mmol) L-Cysteinmethylester-hydrochlorid und 1.35 g Natriumacetat eingetragen. Nach weiteren 3 h bei 100 °C wurde das Xylol abdestilliert, der Rückstand in Dichlormethan aufgenommen und mit $NaHCO_3$-Lsg. sowie Wasser gewaschen. Der Ester wurde durch Entfernen des Lösungsmittels isoliert und ohne weitere Reinigung in die nächste Reaktion eingesetzt.
$[\alpha]_D$ = -57 °C/c = 1/MeOH)

Beispiel 7

9b-Phenyl-2,3-dihydrothiazolo-[2,3-a]isoindol-5(9bH)-on-3-carbonsäure

Das Rohprodukt der letzten Reaktion wurde in 5 ml Ethanol gelöst, mit 2 ml 2 N NaOH versetzt und 2 h bei 40 °C gerührt.

Dann wurde der Alkohol abdestilliert, die wäßrige Phase mit 6 N HCl angesäuert und die freie Säure abgesaugt. Ausb. 0.6 g (44 % d.Th., bezogen auf eingesetzte 2-Benzoylbenzoesäure), Schmp. 96-98 °C (Umkristallisation aus Ethanol). $[\alpha]_D$ = - 211 °/c = 0.89 /MeOH.

Beispiel 8

9b-Phenyl-2,3-dihydrothiazolo-[2,3-a]isoindol-5(9bH)-on-3-carbonsäuremorpholid

311 mg (1 mmol) 9b-Phenyl-2,3-dihydrothiazolo-[2,3-a]isoindol-5(9bH)-on-3-carbonsäure (Bsp. 7) in 10 ml abs. Dichlormethan wurden bei -15 °C mit 101 mg (1 mmol) 4-Methylmorpholin und anschließend 155 mg (1.1 mmol) Isobutylchlorformiat versetzt und 15 min gerührt. Dann wurden 96 mg (1.1 mmol) Morpholin zugegeben, auf RT erwärmt und 4 h bei RT gerührt. Nach Zugabe weiterer 20 ml Dichlormethan wurde die Lösung mit $NaHCO_3$-Lsg. und Wasser ausgeschüttelt, über $Na_2SO_4$ getrocknet und vom Lösungsmittel befreit. Der Rückstand wurde durch Chromatographie an Kieselgel mit Essigester als Eluens gereinigt. Ausb. 158 mg (51 % d. Th.), Schmp. 138-141 °C.

8.1 9b-Phenyl-2,3-dihydrothiazolo-[2,3-a]isoindol-5(9bH)-on-3-carbonsäureamid
wurde analog zu Bsp. 8 in 57 % Ausbeute hergestellt, Schmp. 164 °C (Essigester).

8.2 9b-Phenyl-2,3-dihydrothiazolo-[2,3-a]isoindol-5(9bH)-on-3-carbonsäuremethylamid
wurde analog zu Bsp. 8 in 41 % Ausbeute hergestellt, Schmp. 160-162 °C (Essigester).

8.3 9b-Phenyl-2,3-dihydrothiazolo-[2,3-a]isoindol-5(9bH)-on-3-carbonsäuredimethylamid
wurde analog zu Bsp. 8 in 59 % Ausbeute hergestellt, Schmp. 178 °C (Essigester).

8.4. 9b-Phenyl-2,3-dihydrothiazolo-[2,3-a]isoindol-5(9bH)-on3-carbonsäurepropylester
wurde analog zu Bsp. 8 durch Umsetzung des Aktivesters mit n-Propanol in 27 % Ausbeute hergestellt, Schmp. 103-106 °C.

8.5 9b-Phenyl-2,3-dihydrothiazolo-[2,3-a]isoindol-5(9bH)-on-3-carbonsäureisonropylester
wurde analog zu Bsp. 8 durch Umsetzung des Aktivesters mit 2-Propanol in 31 % Ausbeute hergestellt, Schmp. 88-90 °C.

8.6 9b-Phenyl-2,3-dihydrothiazolo-[2,3-a]isoindol-5(9bH)-on-3-carbonsäuremethoxyethylester
wurde analog zu Bsp. 8 durch Umsetzung des Aktivesters mit 2-Methoxyethanol in 30 % Ausbeute hergestellt, Öl.

8.7 9b-Phenyl-2,3-dihydrothiazolo-[2,3-a]isoindol-5(9bH)-on-3-carbonsäureisobutylester
wurde analog zu Bsp. 8 durch Umsetzung des Aktivesters mit 2-Methyl-1-propanol in 42 % Ausbeute hergestellt, Öl.

8.8 9b-Phenyl-2,3-dihydrothiazolo-[2,3-a]isoindol-5(9bH)-on-3-carbonsäure-(2-pyridylmethyl)ester
wurde analog zu Bsp. 8 durch Umsetzung des Aktivesters mit 2-(Hydroxymethyl)pyridin in 42 % Ausbeute hergestellt.

8.9 9b-Phenyl-2,3-dihydrothiazolo-[2,3-a]isoindol-5(9bH)-on-3-carbonsäure-(3-pyridylmethyl)ester
wurde analog zu Bsp. 8 durch Umsetzung des Aktivesters mit 3-(Hydroxymethyl)pyridin in 60 % Ausbeute hergestellt, Schmp. 119-121 °C.
8.10 9b-Phenyl-2,3-dihydrothiazolo-[2,3-a]isoindol-5(9bH)-on-3-carbonsäure-(4-pyridylmethyl)ester
wurde analog zu Bsp. 8 durch Umsetzung des Aktivesters mit 4-(Hydroxymethyl)pyridin in 37 % Ausbeute hergestellt, Schmp. 125-128 °C.

Beispiel 9

Hemmung der Reversen Transkriptase (RT) durch Derivate des 9b-Phenyl-2,3-dihydro-thiazolo-[2,3-a]-isoindol-5(9bH)-on

Das Screeningtestsystem beinhaltet die gereinigte RT aus HIV-1, die durch gentechnologische Methoden in E. coli exprimiert wurde, sowie die Komponenten des Initiationskomplexes, wie die in-vitro-Transkripte des HIV-LTR's mit der benachbarten Primer Binding Site als Template und einem zur Primer Binding Site komplementären 18mer Oligonukleotid als Primer. Gemessen wurde der [$^3$H]-Thymidin-5'-triphosphat-Einbau durch Auszählen im $\beta$-Counter. In der folgenden Tabelle wird für die untersuchten Verbindungen der IC50-Wert angegeben. Dieser Wert entspricht derjenigen Konzentration der Testsubstanz, die eine Hemmung der Reversen Transkriptase Aktivität um 50% bewirkt. Als Vergleichssubstanz wurde der Wert für AZT entsprechend bestimmt.

Ergebnisse:

| Substanz | Hemmung der HIV-RT $IC_{50}$[M] |
|---|---|
| 3'-Azido-3'-desoxythymidin-5'-triphosphat [AZT-TP] | $6.0 \times 10^{-6}$ |
| 9b-Phenyl-2,3-dihydrothiazolo-[2,3-a]isoindol-5(9bH)-thion | $3.5 \times 10^{-6}$ |
| 7,8-Dichlor-9b-phenyl-2,3-dihydrothiazolo-[2,3,-a]isoindol-5(9bH)-on | $4.5 \times 10^{-6}$ |
| 9b-(2-Thienyl)-2,3-dihydrothiazolo-[2,3,-a]isoindol-5(9bH)-on | $2.7 \times 10^{-6}$ |
| 9b-(2-Furyl)-2,3-dihydrothiazolo-[2,3,-a]isoindol-5(9bH)-on | $1.4 \times 10^{-6}$ |

**Patentansprüche**

1. Verwendung von Thiazolo-[2,3-a]isoindol-Derivate der Formel I

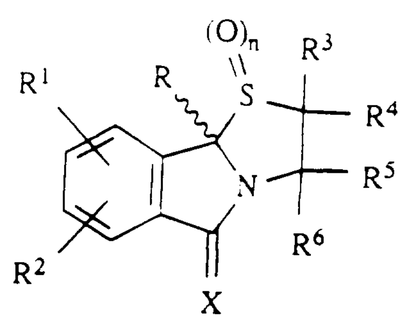

(I),

zur Herstellung von Arzneimitteln mit antiviraler Wirkung, wobei
X ein Sauerstoff- oder Schwefelatom, die Iminogruppe =NH oder eine N-$C_1$-$C_5$-Alkyliminogruppe sein kann,
n gleich 0, 1 oder 2 ist,
R ein Wasserstoffatom, einen geradkettigen oder verzweigten, gesättigten oder ungesättigten aliphatischen Rest mit 1-9 C-Atomen, der durch Phenyl substituiert sein kann, oder einen $C_1$-$C_6$-Alkoxy-$C_1$-$C_6$-alkyl- oder $C_1$-$C_6$-Alkylmercapto-$C_1$-$C_6$-alkylrest bedeutet, oder

einen Phenylring bedeutet, der gegebenenfalls ein- oder mehrfach substituiert ist durch $C_1$-$C_6$-Alkyl, $C_1$-$C_6$-Alkoxy, $C_1$-$C_6$-Alkylmercapto, $C_1$-$C_6$-Alkylsulfinyl, $C_1$-$C_6$-Alkylsulfonyl, $C_2$-$C_6$-Alkenyl, $C_2$-$C_6$-Alkinyl, $C_2$-$C_6$-Alkenyloxy, $C_2$-$C_6$-Alkenylmercapto, $C_2$-$C_6$-Alkinyloxy, $C_2$-$C_6$-Alkinylmercapto, Amino, $C_1$-$C_6$-Alkylamino, Di-$C_1$-$C_6$-alkylamino, $C_1$-$C_6$-Alkylcarbonylamino, $C_1$-$C_6$-Alkylaminocarbonyl, $C_1$-$C_6$-Alkoxycarbonyl, Aminocarbonyl, Hydroxy, Benzyloxy, Phenylmercapto, Phenyloxy, Nitro, Cyano, Halogen, Trifluormethyl, Azido, Formylamino, Carboxy oder Phenyl, oder
einen mono-, bi- oder tricyclischen carbocyclischen Ring mit 7-15 C-Atomen oder ein heterocyclisches mono-, bi- oder tricyclisches Ringsystem mit jeweils 5 oder 6 Ringatomen bedeutet und pro Ringsystem 1-4 bzw. 1-5 Heteroatome enthalten sein können, wobei die Heteroatome Stickstoff, Schwefel oder Sauerstoff sind, und diese Ringe durch $C_1$-$C_6$-Alkyl, $C_1$-$C_6$-Alkoxy, Nitro, Amino oder Halogen substituiert sein können,
$R^1$ ein Wasserstoffatom, einen geradkettigen oder verzweigten, gesättigten oder ungesättigten aliphatischen Rest mit 1-6 C-Atomen oder $C_1$-$C_6$-Alkoxy, $C_1$-$C_6$-Alkylmercapto, $C_1$-$C_6$-Alkylsulfinyl, $C_1$-$C_6$-Alkylsulfonyl, Amino, $C_1$-$C_6$-Alkylamino, Di-$C_1$-$C_6$-Alkylamino, Sulfonamido, $C_1$-$C_6$-Alkoxycarbonyl, Carboxy, Halogen, Hydroxy, Nitro, Cyano, Azido, Phenyl oder Benzyloxy bedeutet,
$R^2$ die gleiche Bedeutung hat wie $R^1$, wobei die Reste $R^1$ und $R^2$ unabhängig voneinander gleich oder verschieden sein können,
$R^3$ Wasserstoff, $C_1$-$C_6$-Alkyl, $C_1$-$C_6$-Alkoxy, $C_1$-$C_6$-Alkylmercapto, Amino, $C_1$-$C_6$-Alkylamino, Di-$C_1$-$C_6$-Alkylamino, Aminocarbonyl, $C_1$-$C_6$-Alkylaminocarbonyl, Di-$C_1$-$C_6$-alkylaminocarbonyl, Morpholinocarbonyl, Halogen, Cyano, Hydroxy, Carboxy, $C_1$-$C_6$-Alkoxycarbonyl, Aryloxycarbonyl, Hetaryloxycarbonyl, Aryl-$C_1$-$C_6$-alkoxycarbonyl, Hetaryl-$C_1$-$C_6$-alkoxycarbonyl, $C_1$-$C_6$-Alkoxy-$C_1$-$C_6$-alkoxycarbonyl oder Hydroxy-$C_1$-$C_6$-alkoxycarbonyl bedeutet, wobei die Aryl- oder Hetarylreste jeweils substituiert sein können durch $C_1$-$C_6$-Alkyl, $C_1$-$C_6$-Alkoxy oder Halogen,
$R^4$, $R^5$, $R^6$ die gleiche Bedeutung wie $R^3$ haben, wobei die Reste $R^3$, $R^4$, $R^5$ und $R^6$ unabhängig voneinander gleich oder verschieden sein können,
sowie deren Tautomere, Enantiomere, Diastereomere und physiologisch verträgliche Salze,
mit der Maßgabe, daß für den Fall, daß $R^1$, $R^2$, $R^3$, $R^4$, $R^5$ und $R^6$ gleichzeitig Wasserstoff, n die Zahlen 0 bzw. 1 und X ein Sauerstoffatom bedeuten, der Rest R nicht Wasserstoff, eine aliphatische Gruppe mit 1-7 C-Atomen, die durch Phenyl substituiert sein kann, oder Phenyl, das ein- oder mehrfach substituiert ist durch $C_1$-$C_4$-Alkyl, $C_1$-$C_4$-Alkoxy, Hydroxy, Trifluormethyl, Methylsulfonyl oder Halogen, bedeuten kann.

**2.** Verwendung gemäß Anspruch 1, dadurch gekennzeichnet, daß
R einen carbocyclischen Ring mit 7-15 C-Atomen darstellt ausgewählt aus der Gruppe Naphthyl, Anthracenyl, Phenanthrenyl, Fluorenyl, Indanyl, Indenyl, Acenaphthylenyl, Norbornyl, Adamantyl, $C_3$-$C_7$-Cycloalkyloder $C_5$-$C_8$-Cycloalkenyl.

**3.** Verwendung gemäß Anspruch 1, dadurch gekennzeichnet, daß
R ein heterocyclisches mono-, bi- oder tricyclisches 1-, 2- oder 3-Ringsystem mit jeweils 5 oder 6 Ringatomen bedeutet und pro Ringsystem 1-4 bzw. 1-5 Heteroatome enthalten sein können, wobei die Heteroatome Stickstoff, Schwefel oder Sauerstoff sind, ausgewählt aus der Gruppe Pyridin-, Pyrimidin-, Pyridazin-, Pyrazin- , Triazin-, Pyrrol-, Pyrazol-, Imidazol-, Triazol-, Thiazol-, Oxazol-, Isoxazol-, Oxadiazol-, Furazan-, Furan- , Thiophen-, Indol-, Chinolin-, Isochinolin-, Cumaron-, Thionaphthen-, Benzoxazol-, Benzthiazol-, Indazol-, Benzimidazol-, Benztriazol-, Chromen-, Phthalazin-, Chinazolin-, Chinoxalin-, Methylendioxybenzol-, Carbazol-, Acridin-, Phenoxazin-, Phenothiazin-, Phenazin- oder Purinsystem, wobei die ungesättigten bzw. aromatischen Heterocyclen partiell oder vollständig hydriert sein können.

**4.** Verwendung gemäß Anspruch 1, 2 oder 3, dadurch gekennzeichnet, daß $R^1$ Wasserstoff, $C_1$-$C_6$-Alkyl, $C_1$-$C_6$-Alkoxy, Sulfonamido, Amino, Hydroxy oder Halogen und $R^2$ Wasserstoff oder Halogen bedeutet.

**5.** Verwendung gemäß einem der Ansprüche 1-4, dadurch gekennzeichnet, daß $R^3$ und $R^4$ Wasserstoff bedeuten.

**6.** Verwendung gemäß einem der Ansprüche 1-5, dadurch gekennzeichnet, daß $R^5$ Wasserstoff, $C_1$-$C_6$-Alkyl, Carboxy, $C_1$-$C_6$-Alkoxycarbonyl, Morpholinocarbonyl, Aminocarbonyl, $C_1$-$C_6$-Alkylaminocarbonyl, Di-$C_1$-$C_6$-Alkylaminocarbonyl, $C_1$-$C_6$-Alkoxy-$C_1$-$C_6$-alkoxycarbonyl, Pyridyl-$C_1$-$C_6$-alkoxycarbonyl oder Halogen und $R^6$ Wasserstoff bedeutet.

**7.** Thiazolo-[2,3-a]isoindol-Derivate der allgemeinen Formel I

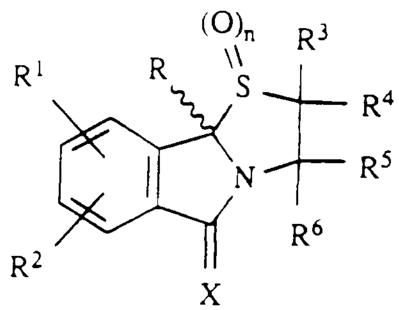

(I),

in der
X ein Sauerstoff- oder Schwefelatom, die Iminogruppe =NH oder eine N-$C_1$-$C_5$-Alkyliminogruppe sein kann,
n gleich 0, 1 oder 2 ist,
R ein heterocyclisches mono-, bi- oder tricyclisches Ringsystem mit jeweils 5 oder 6 Ringatomen bedeutet und pro Ringsystem 1-4 bzw. 1-5 Heteroatome enthalten sein können, wobei die Heteroatome Stickstoff, Schwefel oder Sauerstoff sind, und diese Ringe durch $C_1$-$C_6$-Alkyl, $C_1$-$C_6$-Alkoxy, Nitro, Amino oder Halogen substituiert sein können,
$R^1$ ein Wasserstoffatom, einen geradkettigen oder verzweigten, gesättigten oder ungesättigten aliphatischen Rest mit 1-6 C-Atomen oder $C_1$-$C_6$-Alkoxy, $C_1$-$C_6$-Alkylmercapto, $C_1$-$C_6$-Alkylsulfinyl, $C_1$-$C_6$-Alkylsulfonyl, Amino, $C_1$-$C_6$-Alkylamino, Di-$C_1$-$C_6$-Alkylamino, Sulfonamido, $C_1$-$C_6$-Alkoxycarbonyl, Carboxy, Halogen, Hydroxy, Nitro, Cyano, Azido, Phenyl oder Benzyloxy bedeutet,
$R^2$ die gleiche Bedeutung hat wie $R^1$, wobei die Reste $R^1$ und $R^2$ unabhängig voneinander gleich oder verschieden sein können,
$R^3$ Wasserstoff, $C_1$-$C_6$-Alkyl, $C_1$-$C_6$-Alkoxy, $C_1$-$C_6$-Alkylmercapto, Amino, $C_1$-$C_6$-Alkylamino, Di-$C_1$-$C_6$-Alkylamino, Aminocarbonyl, $C_1$-$C_6$-Alkylaminocarbonyl, Di-$C_1$-$C_6$-alkylaminocarbonyl, Morpholinocarbonyl, Halogen, Cyano, Hydroxy, Carboxy, $C_1$-$C_6$-Alkoxycarbonyl, Aryloxycarbonyl, Hetaryloxycarbonyl, Aryl-$C_1$-$C_6$-alkoxycarbonyl, Hetaryl-$C_1$-$C_6$-alkoxycarbonyl, $C_1$-$C_6$-Alkoxy-$C_1$-$C_6$-alkoxycarbonyl oder Hydroxy-$C_1$-$C_6$-alkoxycarbonyl bedeutet, wobei die Aryl- oder Hetarylreste jeweils substituiert sein können durch $C_1$-$C_6$-Alkyl, $C_1$-$C_6$-Alkoxy oder Halogen,
$R^4$, $R^5$, $R^6$ die gleiche Bedeutung wie $R^3$ haben, wobei die Reste $R^3$, $R^4$, $R^5$ und $R^6$ unabhängig voneinander gleich oder verschieden sein können,
sowie deren Tautomere, Enantiomere, Diastereomere und physiologisch verträgliche Salze.

**8.** Thiazolo-[2,3-a]isoindol-Derivate gemäß Anspruch 7, dadurch gekennzeichnet, daß R eine gegebenenfalls durch $C_1$-$C_6$-Alkyl oder Halogen substituierte Thienyl-, Furyl-, Pyridyl-, Thionaphthenyl- oder Indolylgruppe bedeutet.

**9.** Verfahren zur Herstellung von Thiazolo-[2,3-a]isoindol-Derivaten gemäß den Ansprüchen 7 oder 8, dadurch gekennzeichnet, daß man
gegebenenfalls substituierten Benzoesäurederivaten der allgemeinen Formel II

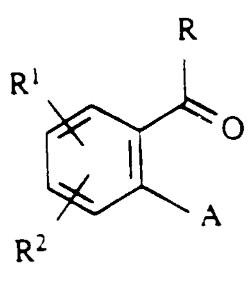

(II)

in der R, $R^1$, und $R^2$ die oben angegebene Bedeutung haben und A gleich -COOH oder C=N ist, mit substituiertem oder unsubstituiertem Cysteamin der allgemeinen Formel III

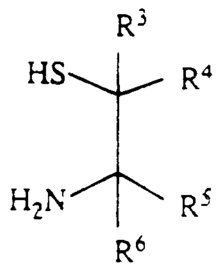

(III)

in der $R^3$, $R^4$, $R^5$ und $R^6$ die oben angegebene Bedeutung haben, in einem geeigneten inerten Lösungsmittel bei Raumtemperatur bis Rückflußtemperatur evtl. in Gegenwart katalytischer Mengen Säuren umsetzt, Verbindungen der Formel I isoliert und gegebenenfalls anschließend Verbindungen der Formel I in andere Verbindungen der Formel I umsetzt und die erhaltenen Racemate gegebenenfalls in ihre optisch aktive Formen auftrennt.

**10.** Arzneimittel enthaltend mindestens eine Verbindung der Formel I gemäß einem der Ansprüche 7 oder 8.

## Claims

**1.** Use of thiazolo-[2,3-a]-isoindole derivatives of the formula I

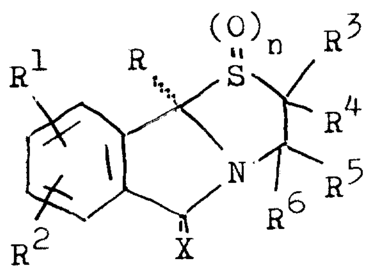

(I)

for the preparation of medicaments with anti-viral action, whereby X can be an oxygen or sulphur atom, the imino group =NH or an N-$C_1$-$C_5$-alkylimino group, n is equal to 0, 1 or 2, R signifies a hydrogen atom, a straight-chained or branched, saturated or unsaturated aliphatic radical with 1 - 9 C-atoms, which can be substituted by phenyl, or a $C_1$-$C_6$-alkoxy-$C_1$-$C_6$-alkyl or $C_1$-$C_6$-alkylmercapto-$C_1$-$C_6$-alkyl radical or signifies a phenyl ring which is possibly substituted one or more times by $C_1$-$C_6$-alkyl, $C_1$-$C_6$-alkoxy, $C_1$-$C_6$-alkylmercapto, $C_1$-$C_6$-alkylsulphinyl, $C_1$-$C_6$-alkylsulphonyl, $C_2$-$C_6$-alkenyl, $C_2$-$C_6$-alkynyl, $C_2$-$C_6$-alkenyloxy, $C_2$-$C_6$-alkenylmercapto, $C_2$-$C_6$-alkynyloxy, $C_2$-$C_6$-alkynylmercapto, amino, $C_1$-$C_6$-alkylamino, di-$C_1$-$C_6$-alkylamino, $C_1$-$C_6$-alkylcarbonylamino, $C_1$-$C_6$-alkylaminocarbonyl, $C_1$-$C_6$-alkoxycarbonyl, aminocarbonyl, hydroxyl, benzyloxy, phenylmercapto, phenyloxy, nitro, cyano, halogen, trifluoromethyl, azido, formylamino, carboxyl or phenyl or signifies a mono-, bi- or tricyclic carbocyclic ring with 7 - 15 C-atoms or a heterocyclic mono-, bi or tricyclic ring system with, in each case, 5 or 6 ring atoms and, per ring system, can contain 1 - 4 or 1 - 5 heteroatoms, respectively, whereby the heteroatoms are nitrogen, sulphur or oxygen, and these rings can be substituted by $C_1$-$C_6$-alkyl, $C_1$-$C_6$-alkoxy, nitro, amino or halogen, $R^1$ signifies a hydrogen atom, a straight-chained or branched, saturated or unsaturated aliphatic radical with 1 - 6 C-atoms or $C_1$-$C_6$-alkoxy, $C_1$-$C_6$-alkylmercapto, $C_1$-$C_6$-alkylsulphinyl, $C_1$- $C_6$-alkylsulphonyl, amino, $C_1$-$C_6$-alkylamino, di-$C_1$-$C_6$-alkylamino, sulphonamido, $C_1$-$C_6$-alkoxycarbonyl, carboxyl, halogen, hydroxyl, nitro, cyano, azido, phenyl or benzyloxy, $R^2$ has the same meaning as $R^1$, whereby the radicals $R^1$ and $R^2$, independently of one another, can be the same or different, $R^3$ signifies hydrogen, $C_1$-$C_6$-alkyl,$C_1$-$C_6$-alkoxy, $C_1$-$C_6$-alkylmercapto, amino, $C_1$-$C_6$-alkylamino, di-$C_1$-$C_6$-alkylamino, aminocarbonyl, $C_1$-$C_6$-alkylaminocarbonyl, di-$C_1$-$C_6$-alkylaminocarbonyl, morpholinocarbonyl, halogen, cyano, hydroxy, carboxyl, $C_1$-$C_6$-alkoxycarbonyl, aryloxycarbonyl, hetaryloxycarbonyl, aryl-$C_1$-$C_6$-alkoxycarbonyl, hetaryl-$C_1$-$C_6$-alkoxycarhonyl, $C_1$-$C_6$-alkoxy-$C_1$-$C_6$-alkoxycarbonyl or hydroxy-$C_1$-$C_6$-alkoxycarbonyl, whereby the arcyl or hetaryl radicals can, in each case, be substituted by $C_1$-$C_6$-alkyl, $C_1$-$C_6$-alkoxy or halogen, $R^4$, $R^5$, $R^6$ have the same meaning as $R^3$, whereby the radicals $R^3$, $R^4$, $R^5$ and $R^6$, independently of one another, can be the same or

different, as well as their tautomers, enantiomers, diastereomers and physiologically acceptable salts, with the proviso that, for the case that $R^1$, $R^2$, $R^3$, $R^4$, $R^5$ and $R^6$ simultaneously signify hydrogen, n the numbers 0 or 1 and X an oxygen atom, the radical R cannot signify hydrogen, an aliphatic group with 1 - 7 C-atoms, which can be substituted by phenyl, or phenyl which is substituted one or more times by $C_1$-$C_4$-alkyl, $C_1$-$C_4$-alkoxy, hydroxyl, trifluoromethyl, methylsulphonyl or halogen

**2.** Use according to claim 1, characterised in that R represents a carbocyclic ring with 7 - 15 C-atoms selected from the group naphthyl, anthracenyl, phenanthrenyl, fluorenyl, indanyl, indenyl, acenaphthenyl, norbornyl, adamantyl, $C_3$-$C_7$-cycloalkyl or $C_5$-$C_8$-cycloalkenyl.

**3.** Use according to claim 1, characterised in that R signifies a heterocyclic mono-, bi- or tricyclic 1-, 2- or 3-ring system which, in each case, 5 or 6 ring atoms and, per ring system, can contain 1 - 4 or 1 - 5 heteroatoms, respectively, whereby the heteroatoms are nitrogen, sulphur or oxygen, selected from the group pyridine, pyrimidine, pyridazine, pyrazine, triazine, pyrrole, pyrazole, imidazole, triazole, thiazole, oxazole, isoxazole, oxadiazole, furazane, furan, thiophene, indole, quinoline, isoquinoline, cumarone, thionaphthene, benzoxazole, benzthiazole, indazole, benzimidazole, benztriazole, chromene, phthalazine, quinazoline, quinoxaline, methylenedioxy-benzene, carbazole, acridine, phenoxazine, phenotniazine, phenazine or purine system, whereby the unsaturated or aromatic heterocycles can be partly or completely hydrogenated.

**4.** Use according to claim 1, 2 or 3, characterised in that $R^1$ signifies hydrogen, $C_1$-$C_6$-alkyl, $C_1$-$C_6$-alkoxy, sulphonamido, amino, hydroxyl or halogen and $R^2$ hydrogen or halogen.

**5.** Use according to one of claims 1 - 4, characterised in that $R^3$ and $R^4$ signify hydrogen.

**6.** Use according to one of claims 1 - 5, characterised in that $R^5$ signifies hydrogen, $C_1$-$C_6$-alkyl, carboxyl, $C_1$-$C_6$-alkoxycarbonyl, morpholinocarbonyl, aminocarbonyl, $C_1$-$C_6$-alkylaminocarbonyl, di-$C_1$-$C_6$-alkylaminocarbonyl, $C_1$-$C_6$-alkoxy-$C_1$-$C_6$-alkoxycarbonyl, pyridyl-$C_1$-$C_6$-alkoxycarbonyl or halogen and $R^6$ hydrogen.

**7.** Thiazolo-[2,3-a]-isoindole derivatives of the general formula I

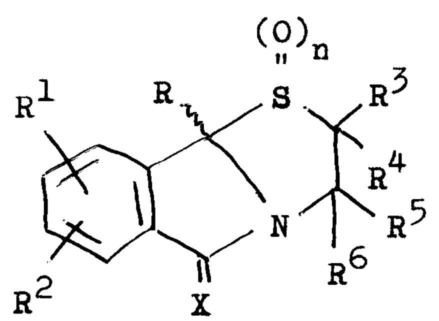

(I)

in which X can be an oxygen or sulphur atom, the imino group =NH or an N-$C_1$-$C_5$-alkylimino radical, n is equal to 0, 1 or 2, R signifies a heterocyclic mono-, bi- or tricyclic ring system with, in each case, 5 or 6 ring atoms and, per ring system, can contain 1 - 4 or 1 - 5 heteroatoms, respectively, whereby the heteroatoms are nitrogen, sulphur or oxygen, and these rings can be substituted by $C_1$-$C_6$-alkyl, $C_1$-$C_6$-alkoxy, nitro, amino or halogen, $R^1$ signifies a hydrogen atom, a straight-chained or branched, saturated or unsaturated aliphatic radical with 1 - 6 C-atoms or $C_1$-$C_6$-alkoxy,$C_1$-$C_6$-alkylmercapto, $C_1$-$C_6$-alkylsulphinyl, $C_1$-$C_6$-alkylsulphonyl, amino, $C_1$-$C_6$-alkylamino, di-$C_1$-$C_6$-alkylamino, sulphonamido, $C_1$-$C_6$-alkoxycarbonyl, carboxyl, halogen, hydroxyl, nitro, cyano, azido, phenyl or benzyloxy, $R^2$ has the same meaning as $R^1$, whereby the radicals $R^1$ and $R^2$, independently of one another, can be the same or different, $R^3$ signifies hydrogen, $C_1$-$C_6$-alkyl, $C_1$-$C_6$-alkoxy, $C_1$-$C_6$-alkylmercapto, amino, $C_1$-$C_6$-alkylamino, di-$C_1$-$C_6$-alkylamino, aminocarbonyl, $C_1$-$C_6$-alkylaminocarbonyl, di-$C_1$-$C_6$-alkylaminocarbonyl, morpholinocarbonyl, halogen, cyano, hydroxyl, carboxyl, $C_1$-$C_6$-alkoxycarbonyl, aryloxycarbonyl, hetaryloxycarbonyl, aryl-$C_1$-$C_6$-alkoxycarbonyl, hetaryl-$C_1$-$C_6$-alkoxycarbonyl, $C_1$-$C_6$-alkoxy-$C_1$-$C_6$-alkoxycarbonyl or hydroxyl-$C_1$-$C_6$-alkoxycarbonyl, whereby the aryl and hetaryl radicals can, in each

case, be substituted by $C_1$-$C_6$-alkyl, $C_1$-$C_6$-alkoxy or halogen, $R^4$, $R^5$, $R^6$ have the same meaning as $R^3$, whereby the radicals $R^3$,$R^4$, $R^5$ and $R^6$, independently of one another, can be the same or different, as well as their tautomers, enantiomers, diastereomers and physiologically acceptable salts.

**8.** Thiazolo-[2,3-a]-isoindole derivatives according to claim 7, characterised in that R signifies a thienyl, furyl, pyridyl, thionaphthenyl or indolyl group possibly substituted by $C_1$-$C_6$-alkyl or halogen.

**9.** Process for the preparation of thiazolo-[2,3-a]-isoindole derivatives according to claims 7 or 8, characterised in that one reacts a possibly substituted benzoic acid derivative of the general formula II

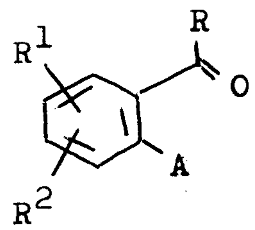

(II)

in which R, $R^1$ and $R^2$ have the above-given meaning and A is equal to -COOH or C=N, with substituted or unsubstituted cysteamine of the general formula III

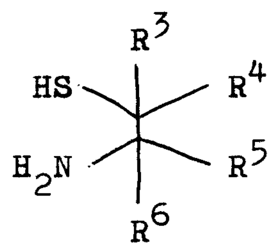

(III)

in which $R^3$, $R^4$, $R^5$ and $R^6$ have the above-given meaning, in a suitable inert solvent at room temperature to reflux temperature, possibly in the presence of a catalytic amount of acids, isolates compounds of the formula I and possibly reacts compounds of the formula I to other compounds of the formula I and possibly separates the racemates obtained into their optically-active forms.

**10.** Medicaments containing at least one compound of the formula I according to one of claims 7 or 8.

**Revendications**

**1.** Utilisation de dérivés de thiazolo-[2,3-a]-isoindol de formule I

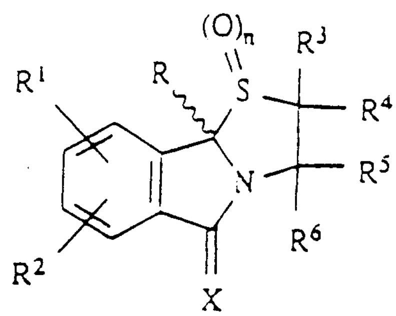

(I),

pour la préparation de médicaments ayant une activité antivirale, dans laquelle

X peut représenter un atome d'oxygène ou de soufre, le groupe imino = NH ou un groupe N-alkyl($C_1$-$C_5$)imino,

n vaut 0, 1 ou 2,

R représente un atome d'hydrogène, un reste aliphatique à chaîne linéaire ou ramifiée, saturée ou insaturée, ayant 1-9 atomes de carbone, qui peut être substitué par un groupe phényle, ou un reste alcoxy($C_1$-$C_6$)-alkyl($C_1$-$C_6$) ou alkyl($C_1$-$C_6$)-mercapto-alkyl($C_1$-$6_6$) ou
un noyau phényle, qui peut être éventuellement substitué une ou plusieurs fois par un groupe alkyle en $C_1$-$C_6$, alcoxy en $C_1$-$C_6$, alkylmercapto en $C_1$-$C_6$, alkylsulfinyle en $C_1$-$C_6$, alkylsulfonyle en $C_1$-$C_6$,alcényle en $C_2$-$C_6$, alcinyle en $C_2$-$C_6$, alcényloxy en $C_2$-$C_6$, alcénylmercapto en $C_2$-$C_6$, alcinyloxy en $C_2$-$C_6$, alcinylmercapto en $C_2$-$C_6$, amino, alkylamino en $C_1$-$C_6$, di(alkyl en $C_1$-$C_6$)amino, alkyl($C_1$-$C_6$)-carbonylamino, alkyl($C_1$-$C_6$)-aminocarbonyle, alcoxy-($C_1$-$C_6$)-carbonyle, aminocarbonyle, hydroxy, benzyloxy, phénylmercapto, phényloxy, nitro, cyano, halogéno, trifluorométhyle, azido, formylamino, carboxy ou phényle, ou
un système de noyaux carbocycliques, mono-, bi- ou tricyclique, ayant 7-15 atomes de C, ou un système annulaire hétérocyclique, mono-, bi- ou tricyclique ayant chaque fois 5 ou 6 chaînons, et pouvant contenir, par système annulaire, 1-4 ou 1-5 hétéroatomes, les hétéroatomes étant de l'azote, du soufre ou de l'oxygène, et ces cycles pouvant être substitués par un groupe alkyle en $C_1$-$C_6$, alcoxy en $C_1$-$C_6$, nitro, amino ou halogéno,

$R^1$ représente un atome d'hydrogène, un reste aliphatique à chaîne linéaire ou ramifiée, saturée ou insaturée, ayant 1-6 atomes de carbone, ou un reste alcoxy en $C_1$-$C_6$, alkylmercapto en $C_1$-$C_6$, alkylsulfinyle en $C_1$-$C_6$,alkylsulfonyle en $C_1$-$C_6$, amino, alkylamino en $C_1$-$C_6$, di(alkyl en $C_1$-$C_6$)amino, sulfonamido, alcoxy-($C_1$-$C_6$)-carbonyle, carboxy, halogéno, hydroxy, nitro, cyano, azido, phényle ou benzyloxy,

$R^2$ a la même signification que $R^1$, les restes $R^1$ et $R^2$ pouvant être indépendamment identiques ou différents,

$R^3$ représente un atome d'hydrogène, un groupe alkyle en $C_1$-$C_6$, alcoxy en $C_1$-$C_6$, alkylmercapto en $C_1$-$C_6$, amino, alkyl($C_1$-$C_6$)-amino, di(alkyl en $C_1$-$C_6$)-amino, amino-carbonyle, alkyl($C_1$-$C_6$)-aminocarbonyle, di(alkyl en $C_1$-$C_6$)-aminocarbonyle, morpholi-nocarbonyle, halogéno, cyano, hydroxy, carboxy, alcoxy-($C_1$-$C_6$)-carbonyle, aryloxy-carbonyle, hétéroaryloxycarbonyle, aryl-alcoxy-($C_1$-$C_6$)-carbonyle hétéroaryl-alcoxy-($C_1$-$C_6$)-carbonyle, alcoxy($C_1$-$C_6$)-alcoxy($C_1$-$C_6$)-carbonyle ou hydroxy-alcoxy-($C_1$-$C_6$)-carbonyle, les restes aryle ou hétéroaryle pouvant être substitués chacun par un groupe alkyle en $C_1$-$C_6$, alcoxy en $C_1$-$C_6$ ou halogèno,

$R^4$, $R^5$, $R^6$ ont la même signification que $R^3$, les restes $R^3$, $R^4$, $R^5$ et $R^6$ pouvant être indépendamment identiques ou différents,

ainsi que leur tautomères, énantiomères, diastéréoisomères et leurs sels physiologiquement acceptables,
étant entendu que dans le cas où $R^1$ $R^2$, $R^3$, $R^4$, $R^5$ et $R^6$ représentent simultanément l'hydrogène, n vaut 0 ou 1 et X représente un atome d'oxygène, le reste R ne peut pas représenter l'hydrogène, un groupe aliphatique ayant 1-7 atomes de carbone, qui peut être substitué par un groupe phényle, ou un groupe phényle qui est substitué une ou plusieurs fois par un groupe alkyle en $C_1$-$C_4$, alcoxy en $C_1$-$C_4$, hydroxy, trifluorométhyle, méthylsulfonyle ou halogéno.

2. Utilisation selon la revendication 1, caractérisée en ce que R représente un noyau carbocyclique ayant 7-15 atomes de carbone, choisi dans le groupe formé par les noyaux napthyle, anthracényle, phénanthrényle, fluorényle, indanyle, indényle, acénaphtényle, norbornyle, adamantyle, cycloalkyle en $C_3$-$C_7$ ou cycloalcényle en $C_5$-$C_8$.

3. Utilisation selon la revendication 1, caractérisée en ce que R représente un système annulaire hétérocyclique, mono-, bi- ou tricyclique ayant chaque fois 5 ou 6 chaînons, et pouvant contenir, par système annulaire, 1-4 ou 1-5 hétéroatomes, les hétéroatomes étant de l'azote, du soufre ou de l'oxygène, choisi dans le groupe constitué par les systèmes à base de pyridine, pyrimidine, pyridazine, pyrazine, triazine, pyrrol, pyrazole, imidazole, triazole, thiazole, oxazole, isoxazole, oxadiazole, furazane, furane, thiophène, indol, quinoléine, isoquinoléine, coumarone, thionaphtène, benzoxazole, benzothiazole, indazole, benzimidazole, benzotriazole, chromène, phtalazine, quinazoline, quinoxaline, méthylènedioxybenzène, carbazole, acridine, phénoxazine, phénothiazine, phénazine ou purine, les hétérocycles insaturés ou aromatiques pouvant être partiellement ou complètement hydrogénés.

4. Utilisation selon l'une quelconque des revendications 1, 2 ou 3, caractérisée en ce que $R^1$ représente un atome d'hydrogène, un groupe alkyle en $C_1$-$C_6$, alcoxy en $C_1$-$C_6$, sulfonamido, amino, hydroxy ou halogéno, et $R^2$ représente un atome d'hydrogène ou d'halogène.

5. Utilisation selon l'une quelconque des revendications 1-4, carctérisée en ce que $R^3$ et $R^4$ représentent l'hydrogène.

6. Utilisation selon l'une quelconque des revendications 1-5, caractérisée en ce que $R^5$ représente l'hydrogène, un groupe alkyle en $C_1$-$C_6$, carboxy, alcoxy($C_1$-$C_6$)-carbonyle, morpholinocarbonyle, aminocarbonyle, alkyl($C_1$-$C_6$)-aminocarbonyle, di(alkyle en $C_1$-$C_6$)-aminocarbonyle, alcoxy-($C_1$-$C_6$)-alcoxy($C_1$-$C_6$)-carbonyle, pyridyl-alcoxy($C_1$-$C_6$)-carbonyle ou halogéno, et $R^6$ représente l'hydrogène.

7. Dérivés de thiazolo-[2,3-a]-isoindol de formule I

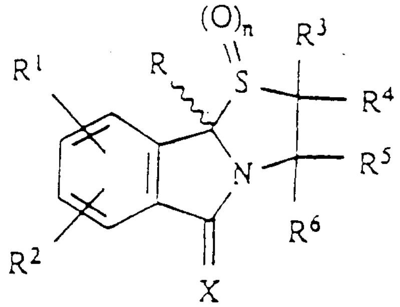

(I),

dans laquelle

| | |
|---|---|
| X | représente un atome d'oxygène ou de soufre, le groupe imino = NH ou un groupe N-alkyl($C_1$-$C_5$)imino, |
| n | vaut 0, 1 ou 2, |
| R | représente un système annulaire hétérocyclique, mono-, bi,- ou tricyclique ayant chaque fois 5 ou 6 chaînons, et pouvant contenir, par système annulaire, 1-4 ou 1-5 hétéroatomes, les hétéroatomes étant de l'azote, du soufre ou de l'oxygène, et ces cycles pouvant être substitués par des groupes alkyle en $C_1$-$C_6$, alcoxy en $C_1$-$C_6$, nitro, amino ou halogéno, |
| $R^1$ | représente un atome d'hydrogène, un reste aliphatique à chaîne linéaire ou ramifiée, saturée ou insaturée, ayant 1-6 atomes de carbone, ou un reste alcoxy en $C_1$-$C_6$, alkylmercapto en $C_1$-$C_6$, alkylsulfinyle en $C_1$-$C_6$,alkylsulfonyle en $C_1$-$C_6$, amino, alkylamino en $C_1$-$C_6$, di(alkyl en $C_1$-$C_6$)-amino, sulfonamido, alcoxy-($C_1$-$C_6$)-carbonyle, carboxy, halogéno, hydroxy, nitro, cyano, azido, phényle ou benzyloxy, |
| $R^2$ | a la même signification que $R^1$, les restes $R^1$ et $R^2$ pouvant être indépendamment identiques ou différents, |
| $R^3$ | représente un atome d'hydrogène un groupe alkyle en $C_1$-$C_6$, alcoxy en $C_1$-$C_6$, alkylmercapto en $C_1$-$C_6$, amino, alkyl($C_1$-$C_6$)-amino, di(alkyl en $C_1$-$C_6$)-amino, aminocarbonyle, alkyl($C_1$-$C_6$)-aminocarbonyle, di(alkyl en $C_1$-$C_6$)-aminocarbonyle, morpholinocarbonyle, halogéno, cyano, hydroxy, carboxy, alcoxy-($C_1$-$C_6$)-carbonyle, aryloxycarbonyle, hétéroaryloxycarbonyle, aryl-alcoxy-($C_1$-$C_6$)-carbonyle, hétéroaryl-alcoxy-($C_1$-$C_6$)-carbonyle, alcoxy($C_1$-$C_6$)-alcoxy($C_1$-$C_6$)-carbonyle ou hydroxy-alcoxy-($C_1$-$C_6$)-carbonyle, les restes aryle ou hétéroaryle pouvant être substitués chacun par un groupe alkyle en $C_1$-$C_6$, alcoxy en $C_1$-$C_6$ ou halogéno, |
| $R^4$, $R^5$, $R^6$ | ont la même signification que $R^3$, les restes $R^3$, $R^4$, $R^5$ et $R^6$ pouvant être indépendamment identiques ou différents, |

ainsi que leur tautomères, énantiomères, diastéréoisomères et leurs sels physiologiquement acceptables.

8. Dérivés de thiazolo-[2,3-a]-isoindol selon la revendication 7, caractérisés en ce que R représente un groupe thiényle, furyle, pyridyle, thionaphtényle ou indolyle éventuellement substitué par un groupe

alkyle en $C_1$-$C_6$ ou halogéno.

**9.** Procédé de préparation de dérivés de thiazolo-[2,3-a]-isoindol selon la revendication 7 ou 8, caractérisé en ce que l'on fait réagir des dérivés d'acide benzoïque éventuellement substitués, de formule générale II

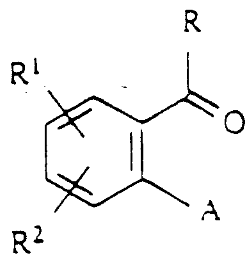

(II)

dans laquelle R, $R^1$ et $R^2$ ont les significations indiquées ci-dessus, et A représente -COOH ou C=N, avec une cystéamine substituée ou non substituée, de formule générale III

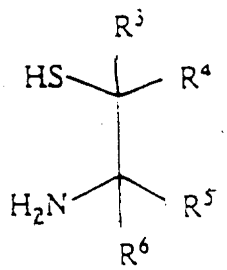

(III)

dans laquelle $R^3$, $R^4$, $R^5$ et $R^6$ ont les significations mentionnées plus haut, dans un solvant inerte approprié, à une température comprise entre la température ambiante et la température de reflux, éventuellement en présence de quantités catalytiques d'acide, on isole les composés de formule I et ensuite, on transforme éventuellement les composés de formule I en d'autres composés de formule I et on sépare éventuellement les racémates obtenus en leurs formes optiquement actives.

**10.** Médicament contenant au moins un composé de formule I selon l'une quelconque des revendications 7 ou 8.